(19) Europäisches Patentamt
European Patent Office
Office européen des brevets

(11) **EP 4 717 260 A2**

(12) **EUROPEAN PATENT APPLICATION**

(43) Date of publication:
**01.04.2026 Bulletin 2026/14**

(21) Application number: **26155796.1**

(22) Date of filing: **03.06.2022**

(51) International Patent Classification (IPC):
***A61K 9/19*** *(2006.01)*

(52) Cooperative Patent Classification (CPC):
**A61K 9/19; A61K 9/0019; A61K 39/39591; A61K 47/10; A61K 47/183; A61K 47/26; A61P 1/00; A61P 1/04; C07K 16/2839;**
A61K 2039/5156

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.06.2021 EP 21177668**

(62) Document number(s) of the earlier application(s) in accordance with Art. 76 EPC:
**22732120.5 / 4 346 899**

(71) Applicant: **Polpharma Biologics S.A.**
**80-172 Gdansk (PL)**

(72) Inventors:
- **JAREMICZ, Zbigniew**
  **80-172 Gdansk (PL)**
- **RIMSA, Vadim**
  **82152 Martinsried (DE)**
- **WURM, Matthias**
  **82152 Martinsried (DE)**

(74) Representative: **Simmons & Simmons LLP (Munich)**
**Lehel Carré**
**Gewürzmühlstraße 11**
**80538 Munich (DE)**

Remarks:
•The complete document including Reference Table(s) and the Sequence Listing(s) can be downloaded from the EPO website
•This application was filed on 02.02.2026 as a divisional application to the application mentioned under INID code 62.
•Claims filed after the date of filing of the application (Rule 68(4) EPC).

(54) **VEDOLIZUMAB FORMULATION**

(57) The present invention is directed to pharmaceutical formulations comprising the anti-α4β7 integrin antibody Vedolizumab. The formulations comprise a specific concentration of trehalose and shows improved handling and stability properties. Furthermore, the present invention concerns products comprising said formulations and uses thereof for the treatment of various diseases.



Processed by Luminess, 75001 PARIS (FR)

## Description

### FIELD OF THE INVENTION

The present invention pertains to the field of antibody formulations, especially formulations comprising anti-$\alpha 4\beta 7$ integrin antibodies, such as Vedolizumab. More specifically, the present invention is directed to lyophilized pharmaceutical formulations comprising the antibody Vedolizumab, as well as related products and uses for the treatment of various diseases and disorders.

### BACKGROUND OF THE INVENTION

Therapeutic preparations comprising proteins, especially antibodies, as active ingredient have become more and more important over the past decades. Most of the therapeutic antibodies are administered via parenteral routes, such as by intravenous infusions or by subcutaneous or intramuscular injection. Stability of the antibody in the formulation is a major concern to guarantee safety and efficacy of the applied therapy. Fine-tuning of the formulation is used to obtain optimal stability, because already small changes in the nature and/or concentration of the ingredients of the formulation influence the antibody's stability.

Vedolizumab is a known therapeutic monoclonal antibody which is used for the treatment of ulcerative colitis and Crohn's disease. It binds to and inhibits integrin $\alpha 4\beta 7$ (LPAM-1) which results anti-inflammatory activity in the gut. Vedolizumab is commonly stored in a lyophilized formulation which is reconstituted prior to administration. The marketed drug product Entyvio comprises a lyophilized Vedolizumab formulation which is reconstituted to consisting of 60 mg/ml antibody, 50 mM histidine, 10 % (w/v) sucrose, 125 mM arginine HCl and 0.06 % (w/v) polysorbate 80 at pH 6.3 (see marketing authorization documents of Entyvio).

There is a need in the art for a lyophilized Vedolizumab formulation with optimized storage and reconstitution abilities.

### SUMMARY OF THE INVENTION

The present invention is based on the finding that the stability the known Vedolizumab formulation used in the marketed product Entyvio can further be increased by optimizing its composition. Especially, replacing sucrose with trehalose and reducing its concentration from 10% (w/v) to about 6 to 8% (w/v) resulted in an improved formulation. The formulation according to the present invention in particular shows a better residual moisture content, a shorter reconstitution time, a better antibody stability upon lyophilization, and a lower tendency for subvisible particle formation.

The present invention therefore provides in a first aspect a liquid pharmaceutical formulation comprising Vedolizumab and trehalose, wherein the concentration of trehalose is between 6% (w/v) and 8% (w/v). Especially, the liquid pharmaceutical formulation comprises Vedolizumab, trehalose, arginine and histidine, wherein the concentration of trehalose is between 6% (w/v) and 8% (w/v).

In a second aspect, the present invention provides a lyophilized pharmaceutical formulation comprising Vedolizumab which can be reconstituted with water to result in a liquid pharmaceutical formulation according to the first aspect.

The present invention further provides in a third aspect a sealed container containing the pharmaceutical formulation according to the first or second aspect, and in a fourth aspect an article of manufacture comprising the container according to the third aspect.

In a fifth aspect, the present invention provides the pharmaceutical formulation according to the first or second aspect or the article of manufacture according to the fourth aspect for use in the treatment of inflammatory bowel disease such as ulcerative colitis and Crohn's disease.

Other objects, features, advantages and aspects of the present invention will become apparent to those skilled in the art from the following description and appended claims. It should be understood, however, that the following description, appended claims, and specific examples, which indicate preferred embodiments of the application, are given by way of illustration only. Various changes and modifications within the spirit and scope of the disclosed invention will become readily apparent to those skilled in the art from reading the following.

### DEFINITIONS

As used herein, the following expressions are generally intended to preferably have the meanings as set forth below, except to the extent that the context in which they are used indicates otherwise.

The expression "comprise", as used herein, besides its literal meaning also includes and specifically refers to the expressions "consist essentially of" and "consist of". Thus, the expression "comprise" refers to embodiments wherein the subject-matter which "comprises" specifically listed elements does not comprise further elements as well as embodiments wherein the subject-matter which "comprises" specifically listed elements may and/or indeed does encompass further elements. Likewise, the expression "have" is to be understood as the expression "comprise", also including and specifically referring to the expressions "consist essentially of" and "consist of". The term "consist essentially of", where possible, in particular refers to embodiments wherein the subject-matter comprises 20% or less, in particular 15% or less, 10% or less or especially 5% or less further elements in addition to the specifically listed elements of which the subject-matter consists essentially of.

The term "about", as used herein, is intended to provide flexibility to a specific value or a numerical range endpoint, providing that a given value may be "a little above" or "a little below" the indicated value accounting for variations one might see in the measurements taken among different instruments, samples, and sample preparations. The term usually means within 5%, and preferably within 1% of a given value or range. The term "about" also includes and specifically refers to the exact indicated number or range.

The term "antibody" in particular refers to a protein comprising at least two heavy chains and two light chains connected by disulfide bonds. Each heavy chain is comprised of a heavy chain variable region (VH) and a heavy chain constant region (CH). Each light chain is comprised of a light chain variable region (VL) and a light chain constant region (CL). The heavy chain-constant region comprises three heavy chain-constant domains (CH1, CH2 and CH3) wherein the first constant domain CH1 is adjacent to the variable region and is connected to the second constant domain CH2 by a hinge region. The light chain-constant region consists only of one constant domain. The variable regions can be further subdivided into regions of hypervariability, termed complementarity determining regions (CDRs), interspersed with regions that are more conserved, termed framework regions (FR), wherein each variable region comprises three CDRs and four FRs. The variable regions of the heavy and light chains contain a binding domain that interacts with an antigen.

The term "pharmaceutical formulation" particularly refers to a composition suitable for administering to a human or animal. Hence, the components of a pharmaceutical formulation are pharmaceutically acceptable.

The terms "lyophilization," or "lyophilized" in particular refer to a process by which the material to be dried is first frozen and then the ice or frozen solvent is removed by sublimation in a vacuum environment. Such technologies are well-known in the art and therefore, are not described in detail herein.

A "reconstituted formulation," as generally used herein, refers to a formulation which has been prepared by dissolving a dry powder, lyophilized, spray-dried or solvent-precipitated antibody and optionally further components in a solvent, such that the antibody and the optional further components are dissolved or dispersed in solution for administration. The solvent in particular is water.

The term "patient" means according to the invention a human being, a nonhuman primate or another animal, in particular a mammal such as a cow, horse, pig, sheep, goat, dog, cat or a rodent such as a mouse and rat. In a particularly preferred embodiment, the patient is a human being.

## DETAILED DESCRIPTION OF THE INVENTION

Based on stability tests of various different formulations for Vedolizumab, the present inventors have found that the claimed formulations, such as for example formulations comprising about 7.2% (w/v) of the sugar trehalose, have optimal shelf storage stability in lyophilized form. Especially, the pharmaceutical composition has a reduced formation of sub-visible particles and the protein concentration in the pharmaceutical formulation does not decrease as much as for the formulation of the marketed Vedolizumab product. Furthermore, the residual moisture content of the pharmaceutical composition is kept lower using the formulation according to the present invention.

In view of this, the present invention provides in a first aspect a liquid pharmaceutical formulation comprising Vedolizumab and trehalose, wherein the concentration of trehalose is between 6% (w/v) and 8% (w/v). Especially, the liquid pharmaceutical formulation comprises Vedolizumab, trehalose, arginine and histidine, wherein the concentration of trehalose is between 6% (w/v) and 8% (w/v).

In a second aspect, the present invention provides a lyophilized pharmaceutical formulation comprising Vedolizumab which can be reconstituted with water to result in a liquid pharmaceutical formulation according to the first aspect.

### 1. Components of the pharmaceutical formulation

Trehalose is a disaccharide formed by a 1,1-glycosidic bond between two $\alpha$-glucose units. Trehalose may be present in the pharmaceutical formulation or used for its preparation in form of pure trehalose or as trehalose dihydrate, i.e. in complex with two water molecules. The weight amount of trehalose in the pharmaceutical formulation, as indicated herein, always refers to the trehalose molecule as such, i.e. without including the mass of the dihydrate in the calculation of the weight amount. The concentration of trehalose in the liquid pharmaceutical formulation is between 6% (w/v) and 8% (w/v) and may preferably be in the range of 6.5% (w/v) to 8 % (w/v), more preferably in the range of 6.5% (w/v) to 7.5% (w/v), more preferably in the range of 7.0 to 7.5% (w/v), and even more preferably in the range of 7.1 to 7.3% (w/v). Most preferably, the concentration of trehalose in the liquid pharmaceutical formulation is 7.2% (w/v). The concentration of trehalose in the lyophilized pharmaceutical formulation may be between about 35% (w/w) and about 45% (w/w). Preferably, the concentration of trehalose in the lyophilized pharmaceutical composition is about 41% (w/w).

Vedolizumab is a humanized monoclonal IgG1 antibody which specifically binds to the integrin $\alpha 4\beta 7$ dimer. Vedolizumab in particular comprises a heavy chain amino acid sequence of SEQ ID NO: 1 and a light chain amino acid sequence of SEQ ID NO: 2. In particular, Vedolizumab is the antibody defined in the WHO Drug Information, Vol. 22, No. 4, 2008 with the International Nonproprietary Name (INN) "Vedolizumab". Vedolizumab may be recombinantly produced in various host cells and suitable cells for recombinant antibody production are known in the art. In one embodiment, Vedolizumab was recombinantly produced in a mammalian cell. Suitable mammalian cells are known in the art and comprise rodent as well as human cell lines. In one embodiment, Vedolizumab was recombinantly produced in a hamster cell. In one embodiment, Vedolizumab was recombinantly produced in a CHO cell.

In certain embodiments, the concentration of Vedolizumab in the liquid pharmaceutical formulation is in the range of 50 to 70 mg/ml, preferably about 60 mg/ml. The concentration of Vedolizumab in the lyophilized pharmaceutical formulation may be between about 30% (w/w) and about 40% (w/w). Preferably, the concentration of Vedolizumab in the lyophilized pharmaceutical composition is about 34% (w/w).

Arginine may be present in the pharmaceutical formulation or used for its preparation in form of the free base or as acid addition salt. In certain embodiments, arginine is present or used as arginine HCl. In particular, the L-enantiomer of arginine is used, i.e. L-arginine. In certain embodiments, the concentration of arginine in the liquid pharmaceutical formulation is in the range of 100 to 200 mM, preferably about 150 mM. The concentration of arginine in the lyophilized pharmaceutical formulation may be between about 15% (w/w) and about 25% (w/w). Preferably, the concentration of arginine in the lyophilized pharmaceutical composition is about 18% (w/w).

Histidine may be present in the pharmaceutical formulation in form of the free base or as acid addition salt. In particular, histidine is present in the pharmaceutical formulation as a mixture of free base and acid addition salt. In certain embodiments, histidine is used as buffer substance and the ratio of free base and acid addition salt is chosen so as to adjust the pharmaceutical formulation to the desired pH value. In certain embodiments, the acid addition salt of histidine may in particular be histidine HCl. In particular, the L-enantiomer of histidine is used, i.e. L-histidine. In certain embodiments, histidine is present in the pharmaceutical formulation as a mixture of L-histidine and L-histidine HCl. In certain embodiments, the concentration of histidine in the liquid pharmaceutical formulation is in the range of 20 to 100 mM, preferably about 50 mM. The concentration of histidine in the lyophilized pharmaceutical formulation may be between about 4% (w/w) and about 8% (w/w). Preferably, the concentration of histidine in the lyophilized pharmaceutical composition is about 5.8% (w/w).

The liquid pharmaceutical formulation in particular has a neutral to light acidic pH. In certain embodiments, the pH of the liquid pharmaceutical formulation is between 6 and 7. Preferably, the pH of the liquid pharmaceutical formulation is about 6.3.

In certain embodiments, the pharmaceutical formulation further comprises a surfactant. The surfactant may especially be a non-ionic surfactant, for example a polymeric non-ionic surfactant. Suitable examples of surfactants are poloxamers. In certain embodiments, the surfactant is a poloxamer, such as poloxamers 188 and 407, especially poloxamer 188. Poloxamer 188, also known as Pluronic F68, is a nonionic triblock copolymers composed of a central hydrophobic chain of polyoxypropylene flanked by two hydrophilic chains of polyoxyethylene. In particular, poloxamer 188 has an oxyethylene content of about 80% and an average molecular weight of about 8400 Da. In certain embodiments, the concentration of the surfactant in the liquid pharmaceutical formulation is in the range of 0.01 to 1% (w/v). In embodiments wherein the surfactant is poloxamer 188, the concentration of the surfactant in the liquid pharmaceutical formulation is in particular in the range of 0.05 to 0.5% (w/v), preferably about 0.2% (w/v). The concentration of the surfactant in the lyophilized pharmaceutical formulation may be between about 0.1% (w/w) and about 2% (w/w). In embodiments wherein the surfactant is poloxamer 188, the concentration of the surfactant in the lyophilized pharmaceutical formulation is in particular in the range of 0.5 to 1.5% (w/w), preferably about 1.1% (w/w).

In certain embodiments, the pharmaceutical formulation does not comprise any sugar except for trehalose. In further embodiments, the pharmaceutical formulation does not comprise any sugar alcohol.

In specific embodiments, the liquid pharmaceutical formulation is an aqueous pharmaceutical formulation. In specific embodiments, the liquid pharmaceutical formulation consists of Vedolizumab, trehalose, arginine, histidine, a surfactant and water, optionally including suitable counterions.

## 2. Use and preparation of the pharmaceutical formulation

The liquid pharmaceutical formulation is advantageously suitable for parenteral delivery. Parenteral administration includes e.g. subcutaneous, intramuscular, intradermal, intramedullary, intrathecal, direct intraventricular, intravenous, intraperitoneal and intravitreal injections. In one embodiment, the liquid pharmaceutical formulation is an injectable formulation. In certain embodiments, the liquid pharmaceutical formulation is suitable for subcutaneous, intravenous, or intramuscular administration. Preferably, the liquid pharmaceutical formulation is suitable for intravenous injection, especially for intravenous infusion.

The liquid pharmaceutical formulation can be prepared by providing a purified Vedolizumab composition and exchanging the buffer using well-known methods. The buffer exchanging may for example be the last step of a purification process of Vedolizumab. Vedolizumab in the final buffer may be concentrated to a desired concentration or a more concentrated form of the antibody is diluted to achieve the desired concentration. Concentration of the formulation can be carried out by any suitable method. In one aspect, the concentration process can include ultrafiltration.

The liquid pharmaceutical formulation can alternatively be prepared by providing a purified Vedolizumab composition comprising some, but not all of the components described herein, and adding the missing components to the composition. In particular, the purified Vedolizumab composition may be a stock solution having a higher concentration than the final liquid pharmaceutical formulation and the missing components may be present in one or more further stock solution. Upon mixing these stock solutions, the liquid pharmaceutical formulation having the desired concentrations of the components is obtained.

The liquid pharmaceutical formulation can also be prepared by reconstitution of a suitable lyophilized formulation, especially a lyophilized pharmaceutical formulation as described herein. Reconstitution in particular is performed by adding water to the lyophilized formulation.

## 2. Specific embodiments of the pharmaceutical formulation

In specific embodiments, the liquid pharmaceutical formulation comprises 50 to 70 mg/ml Vedolizumab, 6 to 8% (w/v) trehalose, 100 to 200 mM arginine and 20 to 100 mM histidine, and has a pH in the range of 6 to 7.

In specific embodiments, the liquid pharmaceutical formulation comprises about 60 mg/ml Vedolizumab, 6 to 8% (w/v) trehalose, 100 to 200 mM arginine and 20 to 100 mM histidine, and has a pH in the range of 6 to 7.

In specific embodiments, the liquid pharmaceutical formulation comprises 50 to 70 mg/ml Vedolizumab, 6 to 8% (w/v) trehalose, about 150 mM arginine and 20 to 100 mM histidine, and has a pH in the range of 6 to 7.

In specific embodiments, the liquid pharmaceutical formulation comprises 50 to 70 mg/ml Vedolizumab, 6 to 8% (w/v) trehalose, 100 to 200 mM arginine and about 50 mM histidine, and has a pH in the range of 6 to 7.

In specific embodiments, the liquid pharmaceutical formulation comprises 50 to 70 mg/ml Vedolizumab, 6 to 8% (w/v) trehalose, 100 to 200 mM arginine and 20 to 100 mM histidine, and has a pH of about 6.3.

In specific embodiments, the liquid pharmaceutical formulation comprises about 60 mg/ml Vedolizumab, 6 to 8% (w/v) trehalose, about 150 mM arginine and about 50 mM histidine, and has a pH in the range of 6 to 7.

In specific embodiments, the liquid pharmaceutical formulation comprises about 60 mg/ml Vedolizumab, 6 to 8% (w/v) trehalose, about 150 mM arginine and about 50 mM histidine, and has a pH of about 6.3.

In specific embodiments, the liquid pharmaceutical formulation comprises 50 to 70 mg/ml Vedolizumab, 6 to 8% (w/v) trehalose, 100 to 200 mM arginine, 20 to 100 mM histidine and 0.05 to 0.5% (w/v) poloxamer 188, and has a pH in the range of 6 to 7.

In specific embodiments, the liquid pharmaceutical formulation comprises about 60 mg/ml Vedolizumab, 6 to 8% (w/v) trehalose, 100 to 200 mM arginine, 20 to 100 mM histidine and 0.05 to 0.5% (w/v) poloxamer 188, and has a pH in the range of 6 to 7.

In specific embodiments, the liquid pharmaceutical formulation comprises 50 to 70 mg/ml Vedolizumab, 6 to 8% (w/v) trehalose, about 150 mM arginine, 20 to 100 mM histidine and 0.05 to 0.5% (w/v) poloxamer 188, and has a pH in the range of 6 to 7.

In specific embodiments, the liquid pharmaceutical formulation comprises 50 to 70 mg/ml Vedolizumab, 6 to 8% (w/v) trehalose, 100 to 200 mM arginine, about 50 mM histidine and 0.05 to 0.5% (w/v) poloxamer 188, and has a pH in the range of 6 to 7.

In specific embodiments, the liquid pharmaceutical formulation comprises 50 to 70 mg/ml Vedolizumab, 6 to 8% (w/v) trehalose, 100 to 200 mM arginine, 20 to 100 mM histidine and 0.05 to 0.5% (w/v) poloxamer 188, and has a pH of about 6.3.

In specific embodiments, the liquid pharmaceutical formulation comprises about 60 mg/ml Vedolizumab, 6 to 8% (w/v) trehalose, about 150 mM arginine, about 50 mM histidine and 0.05 to 0.5% (w/v) poloxamer 188, and has a pH in the range of 6 to 7.

In specific embodiments, the liquid pharmaceutical formulation comprises about 60 mg/ml Vedolizumab, 6 to 8% (w/v) trehalose, about 150 mM arginine, about 50 mM histidine and 0.05 to 0.5% (w/v) poloxamer 188, and has a pH of about 6.3.

In specific embodiments, the liquid pharmaceutical formulation comprises 50 to 70 mg/ml Vedolizumab, 6 to 8% (w/v) trehalose, 100 to 200 mM arginine, 20 to 100 mM histidine and 0.05 to 0.5% (w/v) poloxamer 188, and has a pH in the range of 6 to 7.

In specific embodiments, the liquid pharmaceutical formulation comprises about 60 mg/ml Vedolizumab, 6 to 8% (w/v) trehalose, 100 to 200 mM arginine, 20 to 100 mM histidine and 0.05 to 0.5% (w/v) poloxamer 188, and has a pH in the range of 6 to 7.

In specific embodiments, the liquid pharmaceutical formulation comprises 50 to 70 mg/ml Vedolizumab, 6 to 8% (w/v) trehalose, about 150 mM arginine, 20 to 100 mM histidine and 0.05 to 0.5% (w/v) poloxamer 188, and has a pH in the range of 6 to 7.

In specific embodiments, the liquid pharmaceutical formulation comprises 50 to 70 mg/ml Vedolizumab, 6 to 8% (w/v) trehalose, 100 to 200 mM arginine, about 50 mM histidine and 0.05 to 0.5% (w/v) poloxamer 188, and has a pH in the range of 6 to 7.

In specific embodiments, the liquid pharmaceutical formulation comprises 50 to 70 mg/ml Vedolizumab, 6 to 8% (w/v) trehalose, 100 to 200 mM arginine, 20 to 100 mM histidine and 0.05 to 0.5% (w/v) poloxamer 188, and has a pH of about 6.3.

In specific embodiments, the liquid pharmaceutical formulation comprises about 60 mg/ml Vedolizumab, 6 to 8% (w/v) trehalose, about 150 mM arginine, about 50 mM histidine and 0.05 to 0.5% (w/v) poloxamer 188, and has a pH in the range of 6 to 7.

In specific embodiments, the liquid pharmaceutical formulation comprises about 60 mg/ml Vedolizumab, 6 to 8% (w/v) trehalose, about 150 mM arginine, about 50 mM histidine and 0.05 to 0.5% (w/v) poloxamer 188, and has a pH of about 6.3.

In specific embodiments, the liquid pharmaceutical formulation comprises 50 to 70 mg/ml Vedolizumab, 6 to 8% (w/v) trehalose, 100 to 200 mM arginine, 20 to 100 mM histidine and about 0.2% (w/v) poloxamer 188, and has a pH in the range of 6 to 7.

In specific embodiments, the liquid pharmaceutical formulation comprises about 60 mg/ml Vedolizumab, 6 to 8% (w/v) trehalose, 100 to 200 mM arginine, 20 to 100 mM histidine and about 0.2% (w/v) poloxamer 188, and has a pH in the range of 6 to 7.

In specific embodiments, the liquid pharmaceutical formulation comprises 50 to 70 mg/ml Vedolizumab, 6 to 8% (w/v) trehalose, about 150 mM arginine, 20 to 100 mM histidine and about 0.2% (w/v) poloxamer 188, and has a pH in the range of 6 to 7.

In specific embodiments, the liquid pharmaceutical formulation comprises 50 to 70 mg/ml Vedolizumab, 6 to 8% (w/v) trehalose, 100 to 200 mM arginine, about 50 mM histidine and about 0.2% (w/v) poloxamer 188, and has a pH in the range of 6 to 7.

In specific embodiments, the liquid pharmaceutical formulation comprises 50 to 70 mg/ml Vedolizumab, 6 to 8% (w/v) trehalose, 100 to 200 mM arginine, 20 to 100 mM histidine and about 0.2% (w/v) poloxamer 188, and has a pH of about 6.3.

In specific embodiments, the liquid pharmaceutical formulation comprises about 60 mg/ml Vedolizumab, 6 to 8% (w/v) trehalose, about 150 mM arginine, about 50 mM histidine and about 0.2% (w/v) poloxamer 188, and has a pH in the range of 6 to 7.

In specific embodiments, the liquid pharmaceutical formulation comprises about 60 mg/ml Vedolizumab, 6 to 8% (w/v) trehalose, about 150 mM arginine, about 50 mM histidine and about 0.2% (w/v) poloxamer 188, and has a pH of about 6.3.

The concentration of trehalose in the above specific embodiments may in particular be 6.5% to 7.5% (w/v), preferably about 7.2% (w/v).

The liquid pharmaceutical formulation may in particular consist of water and the components described in any one of the above specific embodiments, and have the pH described in said specific embodiment. The concentration of trehalose in these embodiments may in particular be 7.0% to 7.5% (w/v), preferably about 7.2% (w/v).

In specific embodiments, the liquid pharmaceutical formulation comprises about 60 mg/ml Vedolizumab, 7.0% to 7.5% (w/v) trehalose, about 150 mM arginine, about 50 mM histidine and about 0.2% (w/v) poloxamer 188, and has a pH of about 6.3.

In specific embodiments, the liquid pharmaceutical formulation comprises about 60 mg/ml Vedolizumab, about 7.2% (w/v) trehalose, about 150 mM arginine, about 50 mM histidine and about 0.2% (w/v) poloxamer 188, and has a pH of about 6.3.

In specific embodiments, the liquid pharmaceutical formulation consists of water, 50 to 70 mg/ml Vedolizumab, 6 to 8% (w/v) trehalose, 100 to 200 mM arginine, 20 to 100 mM histidine and 0.05 to 0.5% (w/v) poloxamer 188, and has a pH in the range of 6 to 7.

In specific embodiments, the liquid pharmaceutical formulation consists of water, about 60 mg/ml Vedolizumab, 6.5% to 7.5% (w/v) trehalose, about 150 mM arginine, about 50 mM histidine and about 0.2% (w/v) poloxamer 188, and has a pH of about 6.3.

In specific embodiments, the liquid pharmaceutical formulation consists of water, about 60 mg/ml Vedolizumab, 7.0% to 7.5% (w/v) trehalose, about 150 mM arginine, about 50 mM histidine and about 0.2% (w/v) poloxamer 188, and has a pH of about 6.3.

In specific embodiments, the liquid pharmaceutical formulation consists of water, about 60 mg/ml Vedolizumab, about 7.2% (w/v) trehalose, about 150 mM arginine, about 50 mM histidine and about 0.2% (w/v) poloxamer 188, and has a pH of about 6.3.

In certain embodiments, the liquid pharmaceutical formulation comprises the following components:

**Table 1:** Specific embodiments of the liquid pharmaceutical formulation

| Vedo [mg/ml] | trehalose [% (w/v)] | arginine [mM] | histidine [mM] | PX 188 [% (w/v)] | pH |
|---|---|---|---|---|---|
| 50 - 70 | 6.5 - 7.5 | 100 - 200 | 20 - 100 | 0.05 - 0.5 | 6-7 |
| 50 - 70 | 7.0 - 7.5 | 100 - 200 | 20 - 100 | 0.05 - 0.5 | 6-7 |
| 50 - 70 | 7.2 | 100 - 200 | 20 - 100 | 0.05 - 0.5 | 6-7 |
| 60 | 6.5 - 7.5 | 100 - 200 | 20 - 100 | 0.05 - 0.5 | 6 - 7 |
| 60 | 7.0 - 7.5 | 100 - 200 | 20 - 100 | 0.05 - 0.5 | 6 - 7 |
| 60 | 7.2 | 100 - 200 | 20 - 100 | 0.05 - 0.5 | 6 - 7 |
| 50 - 70 | 6.5 - 7.5 | 150 | 20 - 100 | 0.05 - 0.5 | 6-7 |
| 50 - 70 | 7.0 - 7.5 | 150 | 20 - 100 | 0.05 - 0.5 | 6-7 |
| 50 - 70 | 7.2 | 150 | 20 - 100 | 0.05 - 0.5 | 6-7 |
| 50 - 70 | 6.5 - 7.5 | 100 - 200 | 50 | 0.05 - 0.5 | 6-7 |
| 50 - 70 | 7.0 - 7.5 | 100 - 200 | 50 | 0.05 - 0.5 | 6-7 |
| 50 - 70 | 7.2 | 100 - 200 | 50 | 0.05 - 0.5 | 6-7 |
| 50 - 70 | 6.5 - 7.5 | 100 - 200 | 20 - 100 | 0.2 | 6-7 |
| 50 - 70 | 7.0 - 7.5 | 100 - 200 | 20 - 100 | 0.2 | 6-7 |
| 50 - 70 | 7.2 | 100 - 200 | 20 - 100 | 0.2 | 6 - 7 |
| 50 - 70 | 6.5 - 7.5 | 100 - 200 | 20 - 100 | 0.05 - 0.5 | 6.3 |
| 50 - 70 | 7.0 - 7.5 | 100 - 200 | 20 - 100 | 0.05 - 0.5 | 6.3 |
| 50 - 70 | 7.2 | 100 - 200 | 20 - 100 | 0.05 - 0.5 | 6.3 |
| 60 | 6.5 - 7.5 | 150 | 20 - 100 | 0.05 - 0.5 | 6 - 7 |
| 60 | 7.0 - 7.5 | 150 | 20 - 100 | 0.05 - 0.5 | 6 - 7 |
| 60 | 7.2 | 150 | 20 - 100 | 0.05 - 0.5 | 6 - 7 |
| 60 | 6.5 - 7.5 | 100 - 200 | 50 | 0.05 - 0.5 | 6 - 7 |
| 60 | 7.0 - 7.5 | 100 - 200 | 50 | 0.05 - 0.5 | 6 - 7 |
| 60 | 7.2 | 100 - 200 | 50 | 0.05 - 0.5 | 6 - 7 |
| 60 | 6.5 - 7.5 | 100 - 200 | 20 - 100 | 0.2 | 6-7 |
| 60 | 7.0 - 7.5 | 100 - 200 | 20 - 100 | 0.2 | 6-7 |
| 60 | 7.2 | 100 - 200 | 20 - 100 | 0.2 | 6 - 7 |
| 60 | 6.5 - 7.5 | 100 - 200 | 20 - 100 | 0.05 - 0.5 | 6.3 |
| 60 | 7.0 - 7.5 | 100 - 200 | 20 - 100 | 0.05 - 0.5 | 6.3 |
| 60 | 7.2 | 100 - 200 | 20 - 100 | 0.05 - 0.5 | 6.3 |
| 50 - 70 | 6.5 - 7.5 | 150 | 50 | 0.05 - 0.5 | 6-7 |
| 50 - 70 | 7.0 - 7.5 | 150 | 50 | 0.05 - 0.5 | 6-7 |
| 50 - 70 | 7.2 | 150 | 50 | 0.05 - 0.5 | 6-7 |
| 50 - 70 | 6.5 - 7.5 | 150 | 20 - 100 | 0.2 | 6-7 |
| 50 - 70 | 7.0 - 7.5 | 150 | 20 - 100 | 0.2 | 6-7 |

(continued)

| Vedo [mg/ml] | trehalose [% (w/v)] | arginine [mM] | histidine [mM] | PX 188 [% (w/v)] | pH |
|---|---|---|---|---|---|
| 50 - 70 | 7.2 | 150 | 20 - 100 | 0.2 | 6 - 7 |
| 50 - 70 | 6.5 - 7.5 | 150 | 20 - 100 | 0.05 - 0.5 | 6.3 |
| 50 - 70 | 7.0 - 7.5 | 150 | 20 - 100 | 0.05 - 0.5 | 6.3 |
| 50 - 70 | 7.2 | 150 | 20 - 100 | 0.05 - 0.5 | 6.3 |
| 50 - 70 | 6.5 - 7.5 | 100 - 200 | 50 | 0.2 | 6-7 |
| 50 - 70 | 7.0 - 7.5 | 100 - 200 | 50 | 0.2 | 6-7 |
| 50 - 70 | 7.2 | 100 - 200 | 50 | 0.2 | 6-7 |
| 50 - 70 | 6.5 - 7.5 | 100 - 200 | 50 | 0.05 - 0.5 | 6.3 |
| 50 - 70 | 7.0 - 7.5 | 100 - 200 | 50 | 0.05 - 0.5 | 6.3 |
| 50 - 70 | 7.2 | 100 - 200 | 50 | 0.05 - 0.5 | 6.3 |
| 50 - 70 | 6.5 - 7.5 | 100 - 200 | 20 - 100 | 0.2 | 6.3 |
| 50 - 70 | 7.0 - 7.5 | 100 - 200 | 20 - 100 | 0.2 | 6.3 |
| 50 - 70 | 7.2 | 100 - 200 | 20 - 100 | 0.2 | 6.3 |
| 60 | 6.5 - 7.5 | 150 | 50 | 0.05 - 0.5 | 6 - 7 |
| 60 | 7.0 - 7.5 | 150 | 50 | 0.05 - 0.5 | 6 - 7 |
| 60 | 7.2 | 150 | 50 | 0.05 - 0.5 | 6 - 7 |
| 60 | 6.5 - 7.5 | 150 | 20 - 100 | 0.2 | 6-7 |
| 60 | 7.0 - 7.5 | 150 | 20 - 100 | 0.2 | 6-7 |
| 60 | 7.2 | 150 | 20 - 100 | 0.2 | 6 - 7 |
| 60 | 6.5 - 7.5 | 150 | 20 - 100 | 0.05 - 0.5 | 6.3 |
| 60 | 7.0 - 7.5 | 150 | 20 - 100 | 0.05 - 0.5 | 6.3 |
| 60 | 7.2 | 150 | 20 - 100 | 0.05 - 0.5 | 6.3 |
| 60 | 6.5 - 7.5 | 100 - 200 | 50 | 0.2 | 6-7 |
| 60 | 7.0 - 7.5 | 100 - 200 | 50 | 0.2 | 6-7 |
| 60 | 7.2 | 100 - 200 | 50 | 0.2 | 6-7 |
| 60 | 6.5 - 7.5 | 100 - 200 | 50 | 0.05 - 0.5 | 6.3 |
| 60 | 7.0 - 7.5 | 100 - 200 | 50 | 0.05 - 0.5 | 6.3 |
| 60 | 7.2 | 100 - 200 | 50 | 0.05 - 0.5 | 6.3 |
| 60 | 6.5 - 7.5 | 100 - 200 | 20 - 100 | 0.2 | 6.3 |
| 60 | 7.0 - 7.5 | 100 - 200 | 20 - 100 | 0.2 | 6.3 |
| 60 | 7.2 | 100 - 200 | 20 - 100 | 0.2 | 6.3 |
| 50 - 70 | 6.5 - 7.5 | 150 | 50 | 0.2 | 6-7 |
| 50 - 70 | 7.0 - 7.5 | 150 | 50 | 0.2 | 6-7 |
| 50 - 70 | 7.2 | 150 | 50 | 0.2 | 6-7 |
| 50 - 70 | 6.5 - 7.5 | 150 | 50 | 0.05 - 0.5 | 6.3 |
| 50 - 70 | 7.0 - 7.5 | 150 | 50 | 0.05 - 0.5 | 6.3 |
| 50 - 70 | 7.2 | 150 | 50 | 0.05 - 0.5 | 6.3 |
| 50 - 70 | 6.5 - 7.5 | 100 - 200 | 50 | 0.2 | 6.3 |
| 50 - 70 | 7.0 - 7.5 | 100 - 200 | 50 | 0.2 | 6.3 |

(continued)

| Vedo [mg/ml] | trehalose [% (w/v)] | arginine [mM] | histidine [mM] | PX 188 [% (w/v)] | pH |
|---|---|---|---|---|---|
| 50 - 70 | 7.2 | 100 - 200 | 50 | 0.2 | 6.3 |
| 60 | 6.5 - 7.5 | 150 | 50 | 0.2 | 6-7 |
| 60 | 7.0 - 7.5 | 150 | 50 | 0.2 | 6-7 |
| 60 | 7.2 | 150 | 50 | 0.2 | 6 - 7 |
| 60 | 6.5 - 7.5 | 150 | 50 | 0.05 - 0.5 | 6.3 |
| 60 | 7.0 - 7.5 | 150 | 50 | 0.05 - 0.5 | 6.3 |
| 60 | 7.2 | 150 | 50 | 0.05 - 0.5 | 6.3 |
| 60 | 6.5 - 7.5 | 150 | 20 - 100 | 0.2 | 6.3 |
| 60 | 7.0 - 7.5 | 150 | 20 - 100 | 0.2 | 6.3 |
| 60 | 7.2 | 150 | 20 - 100 | 0.2 | 6.3 |
| 60 | 6.5 - 7.5 | 100 - 200 | 50 | 0.2 | 6.3 |
| 60 | 7.0 - 7.5 | 100 - 200 | 50 | 0.2 | 6.3 |
| 60 | 7.2 | 100 - 200 | 50 | 0.2 | 6.3 |
| 50 - 70 | 6.5 - 7.5 | 150 | 50 | 0.2 | 6.3 |
| 50 - 70 | 7.0 - 7.5 | 150 | 50 | 0.2 | 6.3 |
| 50 - 70 | 7.2 | 150 | 50 | 0.2 | 6.3 |
| 60 | 6.5 - 7.5 | 150 | 50 | 0.2 | 6.3 |
| 60 | 7.0 - 7.5 | 150 | 50 | 0.2 | 6.3 |
| 60 | 7.2 | 150 | 50 | 0.2 | 6.3 |
| Vedo: Vedolizumab; PX 188: poloxamer 188 | | | | | |

In certain embodiments, the liquid pharmaceutical formulation consists of the following components in addition to water:

**Table 2:** Specific embodiments of the liquid pharmaceutical formulation

| Vedo [mg/ml] | trehalose [% (w/v)] | arginine [mM] | histidine [mM] | PX 188 [% (w/v)] | pH |
|---|---|---|---|---|---|
| 50 - 70 | 6.5 - 7.5 | 100 - 200 | 20 - 100 | 0.05 - 0.5 | 6-7 |
| 50 - 70 | 7.0 - 7.5 | 100 - 200 | 20 - 100 | 0.05 - 0.5 | 6-7 |
| 50 - 70 | 7.2 | 100 - 200 | 20 - 100 | 0.05 - 0.5 | 6-7 |
| 60 | 6.5 - 7.5 | 100 - 200 | 20 - 100 | 0.05 - 0.5 | 6 - 7 |
| 60 | 7.0 - 7.5 | 100 - 200 | 20 - 100 | 0.05 - 0.5 | 6 - 7 |
| 60 | 7.2 | 100 - 200 | 20 - 100 | 0.05 - 0.5 | 6 - 7 |
| 50 - 70 | 6.5 - 7.5 | 150 | 20 - 100 | 0.05 - 0.5 | 6-7 |
| 50 - 70 | 7.0 - 7.5 | 150 | 20 - 100 | 0.05 - 0.5 | 6-7 |
| 50 - 70 | 7.2 | 150 | 20 - 100 | 0.05 - 0.5 | 6-7 |
| 50 - 70 | 6.5 - 7.5 | 100 - 200 | 50 | 0.05 - 0.5 | 6-7 |
| 50 - 70 | 7.0 - 7.5 | 100 - 200 | 50 | 0.05 - 0.5 | 6-7 |
| 50 - 70 | 7.2 | 100 - 200 | 50 | 0.05 - 0.5 | 6-7 |
| 50 - 70 | 6.5 - 7.5 | 100 - 200 | 20 - 100 | 0.2 | 6-7 |
| 50 - 70 | 7.0 - 7.5 | 100 - 200 | 20 - 100 | 0.2 | 6-7 |

(continued)

| Vedo [mg/ml] | trehalose [% (w/v)] | arginine [mM] | histidine [mM] | PX 188 [% (w/v)] | pH |
|---|---|---|---|---|---|
| 50 - 70 | 7.2 | 100 - 200 | 20 - 100 | 0.2 | 6 - 7 |
| 50 - 70 | 6.5 - 7.5 | 100 - 200 | 20 - 100 | 0.05 - 0.5 | 6.3 |
| 50 - 70 | 7.0 - 7.5 | 100 - 200 | 20 - 100 | 0.05 - 0.5 | 6.3 |
| 50 - 70 | 7.2 | 100 - 200 | 20 - 100 | 0.05 - 0.5 | 6.3 |
| 60 | 6.5 - 7.5 | 150 | 20 - 100 | 0.05 - 0.5 | 6 - 7 |
| 60 | 7.0 - 7.5 | 150 | 20 - 100 | 0.05 - 0.5 | 6 - 7 |
| 60 | 7.2 | 150 | 20 - 100 | 0.05 - 0.5 | 6 - 7 |
| 60 | 6.5 - 7.5 | 100 - 200 | 50 | 0.05 - 0.5 | 6 - 7 |
| 60 | 7.0 - 7.5 | 100 - 200 | 50 | 0.05 - 0.5 | 6 - 7 |
| 60 | 7.2 | 100 - 200 | 50 | 0.05 - 0.5 | 6 - 7 |
| 60 | 6.5 - 7.5 | 100 - 200 | 20 - 100 | 0.2 | 6-7 |
| 60 | 7.0 - 7.5 | 100 - 200 | 20 - 100 | 0.2 | 6-7 |
| 60 | 7.2 | 100 - 200 | 20 - 100 | 0.2 | 6 - 7 |
| 60 | 6.5 - 7.5 | 100 - 200 | 20 - 100 | 0.05 - 0.5 | 6.3 |
| 60 | 7.0 - 7.5 | 100 - 200 | 20 - 100 | 0.05 - 0.5 | 6.3 |
| 60 | 7.2 | 100 - 200 | 20 - 100 | 0.05 - 0.5 | 6.3 |
| 50 - 70 | 6.5 - 7.5 | 150 | 50 | 0.05 - 0.5 | 6-7 |
| 50 - 70 | 7.0 - 7.5 | 150 | 50 | 0.05 - 0.5 | 6-7 |
| 50 - 70 | 7.2 | 150 | 50 | 0.05 - 0.5 | 6-7 |
| 50 - 70 | 6.5 - 7.5 | 150 | 20 - 100 | 0.2 | 6-7 |
| 50 - 70 | 7.0 - 7.5 | 150 | 20 - 100 | 0.2 | 6-7 |
| 50 - 70 | 7.2 | 150 | 20 - 100 | 0.2 | 6 - 7 |
| 50 - 70 | 6.5 - 7.5 | 150 | 20 - 100 | 0.05 - 0.5 | 6.3 |
| 50 - 70 | 7.0 - 7.5 | 150 | 20 - 100 | 0.05 - 0.5 | 6.3 |
| 50 - 70 | 7.2 | 150 | 20 - 100 | 0.05 - 0.5 | 6.3 |
| 50 - 70 | 6.5 - 7.5 | 100 - 200 | 50 | 0.2 | 6-7 |
| 50 - 70 | 7.0 - 7.5 | 100 - 200 | 50 | 0.2 | 6-7 |
| 50 - 70 | 7.2 | 100 - 200 | 50 | 0.2 | 6-7 |
| 50 - 70 | 6.5 - 7.5 | 100 - 200 | 50 | 0.05 - 0.5 | 6.3 |
| 50 - 70 | 7.0 - 7.5 | 100 - 200 | 50 | 0.05 - 0.5 | 6.3 |
| 50 - 70 | 7.2 | 100 - 200 | 50 | 0.05 - 0.5 | 6.3 |
| 50 - 70 | 6.5 - 7.5 | 100 - 200 | 20 - 100 | 0.2 | 6.3 |
| 50 - 70 | 7.0 - 7.5 | 100 - 200 | 20 - 100 | 0.2 | 6.3 |
| 50 - 70 | 7.2 | 100 - 200 | 20 - 100 | 0.2 | 6.3 |
| 60 | 6.5 - 7.5 | 150 | 50 | 0.05 - 0.5 | 6 - 7 |
| 60 | 7.0 - 7.5 | 150 | 50 | 0.05 - 0.5 | 6 - 7 |
| 60 | 7.2 | 150 | 50 | 0.05 - 0.5 | 6 - 7 |
| 60 | 6.5 - 7.5 | 150 | 20 - 100 | 0.2 | 6-7 |
| 60 | 7.0 - 7.5 | 150 | 20 - 100 | 0.2 | 6-7 |

(continued)

| Vedo [mg/ml] | trehalose [% (w/v)] | arginine [mM] | histidine [mM] | PX 188 [% (w/v)] | pH |
|---|---|---|---|---|---|
| 60 | 7.2 | 150 | 20 - 100 | 0.2 | 6 - 7 |
| 60 | 6.5 - 7.5 | 150 | 20 - 100 | 0.05 - 0.5 | 6.3 |
| 60 | 7.0 - 7.5 | 150 | 20 - 100 | 0.05 - 0.5 | 6.3 |
| 60 | 7.2 | 150 | 20 - 100 | 0.05 - 0.5 | 6.3 |
| 60 | 6.5 - 7.5 | 100 - 200 | 50 | 0.2 | 6-7 |
| 60 | 7.0 - 7.5 | 100 - 200 | 50 | 0.2 | 6-7 |
| 60 | 7.2 | 100 - 200 | 50 | 0.2 | 6-7 |
| 60 | 6.5 - 7.5 | 100 - 200 | 50 | 0.05 - 0.5 | 6.3 |
| 60 | 7.0 - 7.5 | 100 - 200 | 50 | 0.05 - 0.5 | 6.3 |
| 60 | 7.2 | 100 - 200 | 50 | 0.05 - 0.5 | 6.3 |
| 60 | 6.5 - 7.5 | 100 - 200 | 20 - 100 | 0.2 | 6.3 |
| 60 | 7.0 - 7.5 | 100 - 200 | 20 - 100 | 0.2 | 6.3 |
| 60 | 7.2 | 100 - 200 | 20 - 100 | 0.2 | 6.3 |
| 50 - 70 | 6.5 - 7.5 | 150 | 50 | 0.2 | 6-7 |
| 50 - 70 | 7.0 - 7.5 | 150 | 50 | 0.2 | 6-7 |
| 50 - 70 | 7.2 | 150 | 50 | 0.2 | 6-7 |
| 50 - 70 | 6.5 - 7.5 | 150 | 50 | 0.05 - 0.5 | 6.3 |
| 50 - 70 | 7.0 - 7.5 | 150 | 50 | 0.05 - 0.5 | 6.3 |
| 50 - 70 | 7.2 | 150 | 50 | 0.05 - 0.5 | 6.3 |
| 50 - 70 | 6.5 - 7.5 | 100 - 200 | 50 | 0.2 | 6.3 |
| 50 - 70 | 7.0 - 7.5 | 100 - 200 | 50 | 0.2 | 6.3 |
| 50 - 70 | 7.2 | 100 - 200 | 50 | 0.2 | 6.3 |
| 60 | 6.5 - 7.5 | 150 | 50 | 0.2 | 6-7 |
| 60 | 7.0 - 7.5 | 150 | 50 | 0.2 | 6-7 |
| 60 | 7.2 | 150 | 50 | 0.2 | 6 - 7 |
| 60 | 6.5 - 7.5 | 150 | 50 | 0.05 - 0.5 | 6.3 |
| 60 | 7.0 - 7.5 | 150 | 50 | 0.05 - 0.5 | 6.3 |
| 60 | 7.2 | 150 | 50 | 0.05 - 0.5 | 6.3 |
| 60 | 6.5 - 7.5 | 150 | 20 - 100 | 0.2 | 6.3 |
| 60 | 7.0 - 7.5 | 150 | 20 - 100 | 0.2 | 6.3 |
| 60 | 7.2 | 150 | 20 - 100 | 0.2 | 6.3 |
| 60 | 6.5 - 7.5 | 100 - 200 | 50 | 0.2 | 6.3 |
| 60 | 7.0 - 7.5 | 100 - 200 | 50 | 0.2 | 6.3 |
| 60 | 7.2 | 100 - 200 | 50 | 0.2 | 6.3 |
| 50 - 70 | 6.5 - 7.5 | 150 | 50 | 0.2 | 6.3 |
| 50 - 70 | 7.0 - 7.5 | 150 | 50 | 0.2 | 6.3 |
| 50 - 70 | 7.2 | 150 | 50 | 0.2 | 6.3 |
| 60 | 6.5 - 7.5 | 150 | 50 | 0.2 | 6.3 |
| 60 | 7.0 - 7.5 | 150 | 50 | 0.2 | 6.3 |

(continued)

| Vedo [mg/ml] | trehalose [% (w/v)] | arginine [mM] | histidine [mM] | PX 188 [% (w/v)] | pH |
|---|---|---|---|---|---|
| 60 | 7.2 | 150 | 50 | 0.2 | 6.3 |
| Vedo: Vedolizumab; PX 188: poloxamer 188 | | | | | |

### 4. The lyophilized pharmaceutical formulation

In certain embodiments, the present invention provides a lyophilized pharmaceutical formulation which is obtained by lyophilizing a liquid pharmaceutical formulation as described above.

In specific embodiments, the lyophilized pharmaceutical formulation has a residual moisture content of 2 % (w/w) or less, preferably 1 % (w/w) or less. In certain embodiments, the residual moisture content of the lyophilized pharmaceutical formulation directly after lyophilization is 1 % (w/w) or less, preferably 0.5 % (w/w) or less.

### 5. Products containing the pharmaceutical formulation

The present invention provides in a third aspect a sealed container containing the liquid pharmaceutical formulation according to the first aspect of the present invention or lyophilized pharmaceutical formulation according to the second aspect of the present invention. In certain embodiments, the sealed contained contains the lyophilized pharmaceutical formulation. In specific embodiments, the sealed contained is a vial, especially a glass vial.

The present invention provides in a fourth aspect an article of manufacture comprising the container according to the third aspect. The article of manufacture may further contain instructions for use such as a patient information leaflet.

### 6. Therapeutic uses of the pharmaceutical formulation

In a fifth aspect, the present invention provides the pharmaceutical formulation according to the first or second aspect of the present invention or the article of manufacture according to the fourth aspect of the present invention for use in the treatment of a disease. In specific embodiments, the disease is an inflammatory bowel disease such as ulcerative colitis and Crohn's disease. The patient to be treated especially is a human patient.

The present invention provides a method of treatment of a patient in need thereof, comprising the step of administering to said patient a therapeutically effective amount of the liquid pharmaceutical formulation according to the first aspect of the present invention. In specific embodiments, the disease is an inflammatory bowel disease such as ulcerative colitis and Crohn's disease. The patient to be treated especially is a human patient.

## FIGURES

**Figure 1:** Calculated Vedolizumab concentrations of F1 - F3 before and after lyophilization and after stress conditions.

**Figure 2:** Calculated Vedolizumab concentrations of F1 - F3 before lyophilization (T-liquid), after lyophilization (T0) and after storage at A) 2 - 8 °C B) 25 °C C) 40 °C for up to 6 months.

**Figure 3:** Graphical illustration of residual moisture content (RMC) of F1 - F3 at T0 and after storage for up to 6 months.

**Figure 4:** Reconstitution times of F1 - F3 at T0 and after storage at A) 2 - 8 °C B) 25 °C C) 40 °C up to 6 months.

**Figure 5:** Results of the cumulative particle concentrations before and after lyophilization, and after stress conditions.

## EXAMPLES

***Example 1:*** *Filling procedure*

Sample aliquots of 1.0 ml Vedolizumab formulation were manually filled into washed and heat-sterilized 2R glass type I vials by using a Multipette Xstream equipped with a sterile 10-ml Combitip under LAF conditions. The vials were immediately stoppered, crimped and labeled. In addition, 5 vials per formulation were closed, marked and weighed for calculating the reconstitution volume after lyophilization.

**Table 3:** Overview of the composition of formulations F1 to F3.

| Formulation | Antibody | Buffer | pH | Sugar | Excipient | Surfactant |
|---|---|---|---|---|---|---|
| F1 | 60 mg/ml Vedolizumab | 50 mM histidine | 6.3 | 10% (w/v) sucrose | 125 mM arginine | 0.06% (w/v) PS80 |
| F2 | 60 mg/ml Vedolizumab | 50 mM histidine | 6.3 | 10% (w/v) sucrose | 125 mM arginine | 0.2% (w/v) PX188 |
| F3 | 60 mg/ml Vedolizumab | 50 mM histidine | 6.3 | 7.2% (w/v) trehalose | 150 mM arginine | 0.2% (w/v) PX188 |
| PS80: polysorbate 80; PX188: poloxamer 188 | | | | | | |

Formulation F1 corresponds in essence to the formulation used in the marketed product Entyvio.

***Example 2:*** *Stress / storage conditions of the liquid formulations*

*Freeze-thaw stress*

Freeze-thaw stress (T-FT) was performed in a freezer by 3 x freezing the formulations at -70 °C for 22 h and thawing on the lab bench at room temperature for 2 h (samples were protected from light). After performing 3 cycles, the material was used for analytical characterization.

*Shaking stress*

Shaking stress (T-mech) was performed by using a horizontal IKA shaker (IKA KS 4000, ic control, IKA Werke GmbH, Staufen, Germany). Therefore, the formulations were placed in the shaker in upright position. Shaking was performed with 500 rpm at room temperature for 24 h. During this time, the samples were protected from light. After shaking, the material was used for analytical characterization.

*Accelerated storage conditions*

The formulations were stored in 2R vials (each containing 1.0 ml of sample) at:

(i) 5 °C (Liebherr, Mediline LKPV 6520)

(ii) 25 °C/60% r.h. (climate chamber, Konstant Klimaschrank HPP108, Memmert)

***Example 3:*** *Lyophilization*

*Method description*

Freeze-drying of the formulations was performed on an Epsilon 2-12D (Martin Christ GmbH) pilot scale freeze-dryer. The vacuum during the freeze-drying process was controlled by a capacitance manometer and Pirani gauge. The process parameters of the lyophilization cycles without annealing are shown in Table 4, and with annealing step in Table 5.

**Table 4:** Lyophilization process parameters without annealing step.

| # | Step | Time [hh:mm] | Time [min] | Temperature [°C] | Pressure [mbar] | Total time [h] | Ramp |
|---|---|---|---|---|---|---|---|
| 1 | loading | 00:00 | 0 | 5 | 1,000 | 0 | 0 |
| 2 | freezing | 01:30 | 90 | -50 | 1,000 | 1.5 | 0.5 |
| 3 | freezing | 19:40 | 1,180 | -50 | 1,000 | 21.2 | 0 |
| 4 | primary drying | 00:45 | 45 | -50 | 0.1 | 21.9 | 0 |
| 5 | primary drying | 00:36 | 36 | -32 | 0.1 | 22.5 | 0.5 |
| 6 | primary drying | 90:00:00 | 5,400 | -32 | 0.1 | 112.5 | 0 |

(continued)

| # | Step | Time [hh:mm] | Time [min] | Temperature [°C] | Pressure [mbar] | Total time [h] | Ramp |
|---|---|---|---|---|---|---|---|
| 7 | secondary drying | 01:44 | 104 | 20 | 0.1 | 114.3 | 0.5 |
| 8 | secondary drying | 20:00:00 | 900 | 20 | 0.1 | 129.3 | 0 |
| 9 | aeration (filtered N2) | 00:01* | 1 | 20 | 600 | 129.3 | 0 |
| 10 | stoppering | 00:01* | 1 | 20 | 600 | 129.3 | 0 |
| 11 | aeration (filtered N2) | 00:01* | 1 | 20 | 980 | 129.3 | 0 |
| 12 | storage | 00:01* | 1 | 5 | 980 | 129.3 | 0 |

**Table 4:** Lyophilization process parameters without annealing step.

| # | Step | Time [hh:mm] | Time [min] | Temperature [°C] | Pressure [mbar] | Total time [h] | Ramp |
|---|---|---|---|---|---|---|---|
| 1 | loading | 00:00 | 0 | 5 | 1,000 | 0 | 0 |
| 2 | freezing | 01:30 | 90 | -50 | 1,000 | 1.5 | 0.5 |
| 3 | freezing | 04:00 | 240 | -50 | 1,000 | 5.5 | 0 |
| 4 | annealing | 01:20 | 80 | -10 | 1,000 | 6.8 | 0.5 |
| 5 | annealing | 08:00 | 480 | -10 | 1,000 | 14.8 | 0 |
| 6 | annealing | 01:20 | 80 | -50 | 1,000 | 16.2 | 0.5 |
| 7 | freezing | 05:00 | 300 | -50 | 1,000 | 21.2 | 0 |
| 8 | primary drying | 00:45 | 45 | -50 | 0.1 | 21.9 | 0 |
| 9 | primary drying | 00:36 | 36 | -32 | 0.1 | 22.5 | 0.5 |
| 10 | primary drying | 75:00:00 | 4,500 | -32 | 0.1 | 97.5 | 0 |
| 11 | secondary drying | 01:44 | 104 | 20 | 0.1 | 99.3 | 0.5 |
| 12 | secondary drying | 15:00:00 | 900 | 20 | 0.1 | 114.3 | 0 |
| 13 | aeration (filtered N2) | 00:01* | 1 | 20 | 600 | 114.3 | 0 |
| 14 | stoppering | 00:01* | 1 | 20 | 600 | 114.3 | 0 |
| 15 | aeration (filtered N2) | 00:01* | 1 | 20 | 980 | 114.3 | 0 |
| 16 | storage | 00:01* | 1 | 5 | 980 | 114.3 | 0 |

After filling, the vials were partially stoppered by using 13-mm freeze-drying stoppers. The active samples were placed on center positions on the shelves. Active vials were surrounded by shielding buffer vials (i.e., vials containing formulation buffer 1 (50 mM histidine buffer, 125 mM arginine, 10% (w/v) sucrose, 0.06% (w/v) PS80) to reduce the effect of heat radiation to the active formulation. The samples were placed on three shelfs of the freeze-dryer. The freeze-drying process was run under partial loading by using three shelves of the freeze-dryer.

During the freeze-drying process, product temperature, shelf temperature, condenser temperature and chamber pressure (capacitance manometer and Pirani gauge) were monitored. The product temperature was monitored by Pt100 sensors, which were placed in different vials located at different positions on the shelves.

At the end of the process, the stoppers of the vials were closed in the freeze-dryer under nitrogen atmosphere at a pressure of 600 mbar by lifting the shelves. After stoppering of the vials, the chamber was aerated to atmospheric pressure by using nitrogen, and the samples were removed. The samples were crimp-capped after removal from the freeze-dryer (Dara SX-140-C bench top machine), labeled and stored at the respective storage conditions for further analysis.

During the freeze-drying process the Plexiglas door was covered with a stainless steel shield to reduce heat radiation.

The lyophilized formulations were stored at 2-8°C, 25°C and 40°C, respectively, and samples were obtained directly after lyophilization (T0) and after 1 month (T1M), 2 months (T2M), 3 months (T3M) and 6 months (T6M).

***Example 4:*** *UV spectroscopy for determination of antibody concentration*

*Method description*

UV spectroscopy was carried out with a soloVPE system from C Technologies (Bridgewater) coupled to an Agilent Cary 60 UV/VIS spectrometer (Agilent).

The samples were measured undiluted in disposable plastic vessels and with single-use glass fibrettes which were lowered into the sample solution to variable depths to obtain different path lengths.

*Protein concentration determination*

The absorbance (A) of the samples was recorded at 10 different path lengths (l) which were automatically set by the system within the linear range of the Lambert-Beer law.

The protein concentration (c) was calculated from the slope (m) of the regression line resulting from plotting the absorbance at 280 nm against the path length (see equations below). The measurements were performed at 280 nm, with a scatter correction at 320 nm, and an extinction coefficient of 1.562 ml mg-1 cm-1 at this wavelength was used for calculations.

$$A = \varepsilon_{280nm}^{1mg/ml} \times l \times c \qquad m = \frac{A}{l} \qquad c = \frac{m}{\varepsilon_{280nm}^{1mg/ml}}$$

*SST / performance check*

The performance of the system was assured by a daily 'quick check' testing of the light transmission from the Cary 60 to the soloVPE instrument.

*Results*

The Vedolizumab concentrations determined in the different formulations are presented in Figure 1 and Figure 2.

At T0, the Vedolizumab concentration ranged between 57 - 60 mg/ml. Generally, the protein concentration in F3 was higher compared to F1 and F2.

The protein concentration of formulation F2 showed a drop after 1 month stored at 25°C. However, this decrease was not confirmed at later time points. Therefore, F2_T2M_25°C is considered to be an outlier.

***Example 5:*** *Karl Fischer titration for determination of residual moisture content*

*Method description*

The residual moisture content (RMC) of the freeze-dried products was determined by using the coulometric Karl Fischer titrator Aqua 40.00 (Analytik Jena GmbH), which is equipped with a headspace module. The sample preparation of the freeze-dried product was performed in a glovebox under controlled humidity (≤ 10% r.h.).

For the measurement, about 20-30 mg of freeze-dried product were weighed into 2R glass vials (Schott) and heated to 150 °C (temperature determined with ramp) in the oven connected to the reaction vessel via a tubing system. The evaporated water was transferred into the titration solution and the amount of water was determined. The measurement was performed until no more water evaporation was detectable (baseline was determined by the drift before a measurement series and the stop criteria by the oscillation around the baseline). Water content of the sample was calculated taking the environmental moisture as determined in three blanks (empty vials prepared in the measurement environment and analyzed by using the same method and settings as the sample) into consideration.

*SST / performance check*

As a system suitability test, the water content of a Pure Water Standard (Apura water standard oven 1.0, Merck KGaA) was analyzed. Standards were weighed under controlled humidity (≤ 10% r.h.). Therefore ≥80 mg of the standard was measured in triplicates at 170 °C and a minimum measurement time of 5 min. The mean water content of the standard has to be 0.99 ± 0.03% (environmental moisture was subtracted) to comply with the manufacturer specifications.

*Results*

The measured residual moisture contents (RMC) of the formulations are shown in Figure 3.

The initial RMCs (T0) of all formulations were between 0.2% and 1.7%. The highest RMC was observed for F1, which corresponds to the original formulation of the Vedolizumab product Entyvio, and the lowest in F3. Generally, the RMC of F3 was lower than that of F1 and F2 at all testes time points and conditions.

***Example 6:*** *Reconstitution times of freeze-dried products*

*Method description*

The freeze-dried products were reconstituted under LAF conditions by using the following procedure: 0.9 ml water for injection (WFI) was added to the freeze-dried product (into the center of the vial) by using an Eppendorf Combitip pipette. The vial was carefully slewed (shaking was avoided). In order to determine the reconstitution volume, 5 vials per formulation were weighed (i) in liquid state before lyophilization and (ii) in their freeze-dried state after lyophilization. The difference in weight corresponds to the water loss and was taken as reconstitution volume and was determined as 0.9 ml. The reconstitution time was measured as the time to achieve a full reconstitution of the freeze-dried product after the 0.9 ml of liquid had been added. Moreover, the reconstitution behavior was judged with respect to foaming.

*Results*

The reconstitution times determined in the different formulations are presented in Figure 4. The reconstitution times were ranging between 3 to 12 minutes. In general, the reconstitution times of most of the formulations increased over time, independent of the storage conditions. In general, the reconstitution time of F3 was faster compared to that of F1 and F2. After 6 months of storage, the formulations revealed reconstitution times under 6 min.

***Example 7:*** *Micro-Flow Imaging (MFI) for determination of subvisible particle levels*

*Method description*

MFI measurements were conducted with a MFI-5200 particle analyzer system (ProteinSimple) equipped with a silane-coated high-resolution 100-μm SP3 flow cell.

The system was flushed with 5-10 ml water and the background illumination was subsequently optimized by using the corresponding formulation buffer.

All Vedolizumab samples were analyzed undiluted, if possible. A pre-run volume of 0.33 ml was followed by a sample run of 0.30 ml. Approximately 1,100 images were taken per sample. Between the measurements, the flow cell was cleaned with water. MFI View System Software (MVSS) version 2-R2-6.1.20.1915 was used to perform the measurements and MFI View Analysis Suite (MVAS) software version 1.3.0.1007 was used to analyze the samples.

*SST / performance check*

In order to test system suitability of MFI-5200, a two-step check-up was performed before onset of sample measurements:

1) Testing of cleanliness: water was tested with acceptance criteria of ≤200 particles/ml ≥2 μm and ≤1 particle/measurement ≥10 μm

2) Testing of 5-μm counting standard: COUNT-CAL™ CC05 standard (polystyrene latex beads) with acceptance criterion of 2,430 - 3,630 particles/ml ≥3 μm (Acceptance criterion is calculated as follows: 10% deviation of number of beads as provided by supplier + 10% deviation of system accuracy)

3) Detailed information on CC05 as provided by supplier:

a. Count: 3,000 particles/ml (±10%) ≥3 μm

b. Lot. No.: 244167

c. Supplier: Thermo Scientific

*Results*

The results of Micro-Flow Imaging measurements are shown in Figure 5.

Low subvisible particle levels were observed before lyophilization at T-liquid. After 2 weeks of liquid storage at 2 - 8 °C or 25 °C or after freeze-thaw cycles, particle levels increased significantly for F2 compared to F1 and F3. 5.

After lyophilization (T0), the level of subvisible particles was much lower in F3 compared to F1.

**SEQUENCE LISTING**

SEQ ID NO: 1 - vedolizumab heavy chain

```
QVQLVQSGAE VKKPGASVKV SCKGSGYTFT SYWMHWVRQA PGQRLEWIGE 50
IDPSESNTNY NQKFKGRVTL TVDISASTAY MELSSLRSED TAVYYCARGG 100
YDGWDYAIDY WGQGTLVTVS SASTKGPSVF PLAPSSKSTS GGTAALGCLV 150
KDYFPEPVTV SWNSGALTSG VHTFPAVLQS SGLYSLSSVV TVPSSSLGTQ 200
TYICNVNHKP SNTKVDKKVE PKSCDKTHTC PPCPAPELAG APSVFLFPPK 250
PKDTLMISRT PEVTCVVVDV SHEDPEVKFN WYVDGVEVHN AKTKPREEQY 300
NSTYRVVSVL TVLHQDWLNG KEYKCKVSNK ALPAPIEKTI SKAKGQPREP 350
QVYTLPPSRD ELTKNQVSLT CLVKGFYPSD IAVEWESNGQ PENNYKTTPP 400
VLDSDGSFFL YSKLTVDKSR WQQGNVFSCS VMHEALHNHY TQKSLSLSPG 450
K                                                      451
```

SEQ ID NO: 2 - vedolizumab light chain

```
DVVMTQSPLS LPVTPGEPAS ISCRSSQSLA KSYGNTYLSW YLQKPGQSPQ 50
LLIYGISNRF SGVPDRFSGS GSGTDFTLKI SRVEAEDVGV YYCLQGTHQP 100
YTFGQGTKVE IKRTVAAPSV FIFPPSDEQL KSGTASVVCL LNNFYPREAK 150
VQWKVDNALQ SGNSQESVTE QDSKDSTYSL SSTLTLSKAD YEKHKVYACE 200
VTHQGLSSPV TKSFNRGEC                                   219
```

In the following, preferred embodiments are disclosed in a list of items.

**EMBODIMENTS**

1. A liquid pharmaceutical formulation comprising Vedolizumab and trehalose, wherein the concentration of trehalose is between 6% (w/v) and 8% (w/v).

2. The liquid pharmaceutical formulation according to item 1, further comprising arginine and histidine, wherein the concentration of trehalose is between 6.5% (w/v) and 7.5% (w/v).

3. The liquid pharmaceutical formulation according to item 1 or 2, wherein the concentration of trehalose is about 7.2% (w/v).

4. The liquid pharmaceutical formulation according to any one of items 1 to 3, wherein the concentration of Vedolizumab is about 60 mg/ml.

5. The liquid pharmaceutical formulation according to any one of items 1 to 4, wherein the concentration of arginine is between 100 mM and 200 mM, in particular about 150 mM.

6. The liquid pharmaceutical formulation according to any one of items 1 to 5, wherein arginine is arginine HCl.

7. The liquid pharmaceutical formulation according to any one of items 1 to 6, wherein the concentration of histidine is between 20 mM and 100 mM, in particular about 50 mM.

8. The liquid pharmaceutical formulation according to any one of items 1 to 7, wherein histidine is a mixture of L-histidine and L-histidine HCl.

9. The liquid pharmaceutical formulation according to any one of items 1 to 8, wherein the pH of the liquid pharmaceutical formulation is between 6 and 7, in particular about 6.3.

10. The liquid pharmaceutical formulation according to any one of items 1 to 9, further comprising a surfactant.

11. The liquid pharmaceutical formulation according to item 10, wherein the surfactant is poloxamer 188.

12. The liquid pharmaceutical formulation according to item 11, wherein the concentration of poloxamer 188 is between 0.05 % (w/v) and 0.5 % (w/v), in particular 0.2 % (w/v).

13. The liquid pharmaceutical formulation according to any one of items 1 to 12, wherein the pharmaceutical formulation does not comprise any sugar except for trehalose and does not comprise any sugar alcohol.

14. The liquid pharmaceutical formulation according to any one of items 1 to 13, being an aqueous pharmaceutical formulation.

15. The liquid pharmaceutical formulation according to any one of items 1 to 14, consisting of Vedolizumab, trehalose, arginine, histidine, a surfactant and water.

16. A lyophilized pharmaceutical formulation comprising Vedolizumab which can be reconstituted with water to result in a liquid pharmaceutical formulation according to any one of items 1 to 15.

17. The lyophilized pharmaceutical formulation according to item 16, having a residual moisture content of 2 % (w/w) or less.

18. A sealed container containing the pharmaceutical formulation according to any one of items 1 to 17.

19. An article of manufacture comprising the container according to item 18.

20. The pharmaceutical formulation according to any one of items 1 to 17 or the article of manufacture according to item 19 for use in the treatment of inflammatory bowel disease such as ulcerative colitis and Crohn’s disease.

## Claims

**1.** A liquid pharmaceutical formulation comprising Vedolizumab and sucrose, wherein the concentration of sucrose is between 6% (w/v) and 8% (w/v).

**2.** The liquid pharmaceutical formulation according to claim 1, further comprising arginine and histidine.

**3.** The liquid pharmaceutical formulation according to any one of claims 1 to 4, wherein the concentration of Vedolizumab is in the range of 50 to 70 mg/l.

**4.** The liquid pharmaceutical formulation according to any one of claims 1 to 5, wherein arginine is in the form of a free base, wherein optionally the concentration of arginine is between 100 mM and 200 mM.

**5.** The liquid pharmaceutical formulation according to any one of claims 1 to 6, wherein histidine is in the form of the free base, wherein optionally the concentration of histidine is between 20 mM and 100 mM.

**6.** The liquid pharmaceutical formulation according to any one of claims 1 to 7, further comprising a surfactant.

7. The liquid pharmaceutical formulation according to claim 6, wherein the surfactant is poloxamer 407.

8. The liquid pharmaceutical formulation according to any one of claims 1 to 7, being an aqueous pharmaceutical formulation.

9. A sealed container containing the pharmaceutical formulation according to any one of claims 1 to 8.

10. An article of manufacture comprising the container according to claim 9.

11. The pharmaceutical formulation according to any one of claims 1 to 9 or the article of manufacture according to claim 10 for use in the treatment of inflammatory bowel disease such as ulcerative colitis and Crohn’s disease.

12. The pharmaceutical formulation for use of claim 11 or the article of manufacture for use of claim 11, wherein the treatment comprises subcutaneous administration of the pharmaceutical formulation to a patient in need thereof.

Figure 1

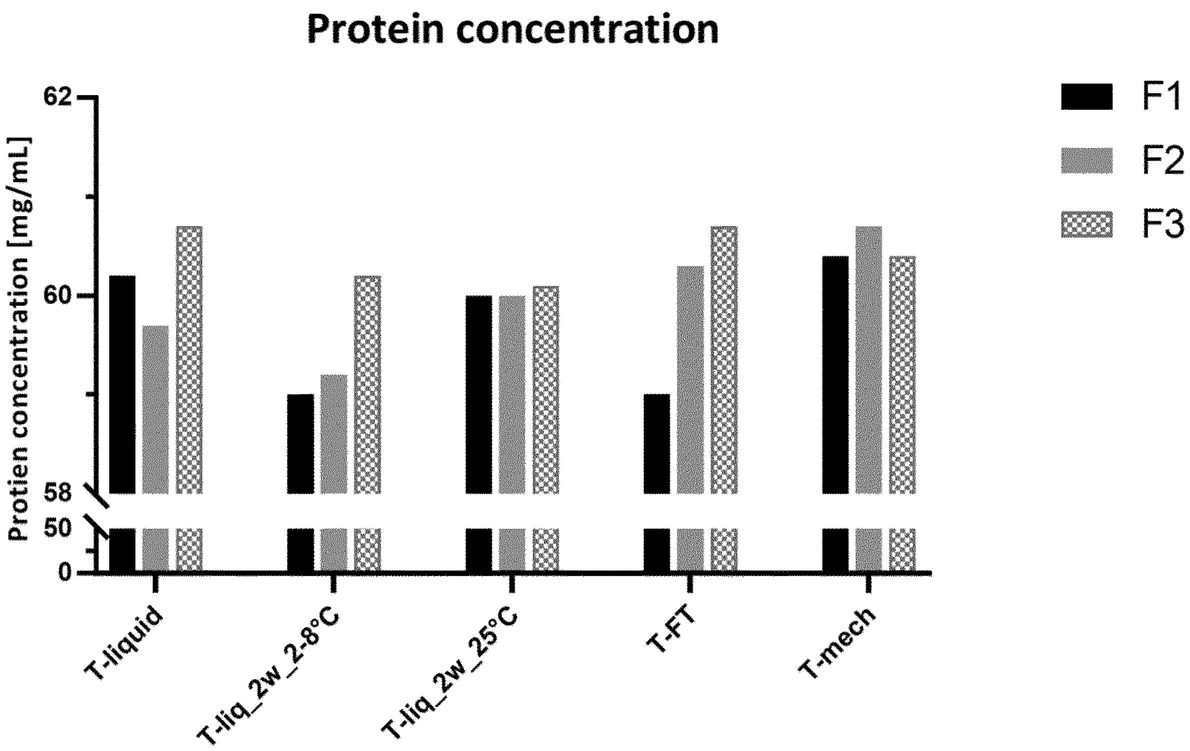


Figure 2

A

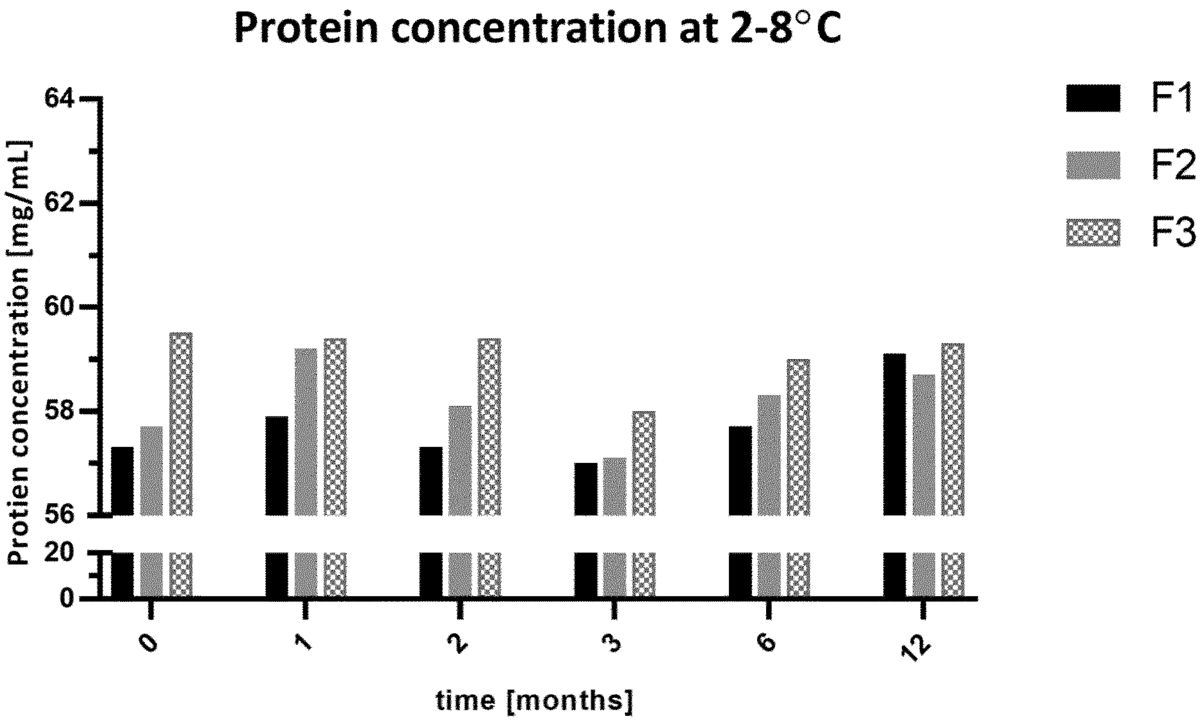

**Figure 2 - continued**

B

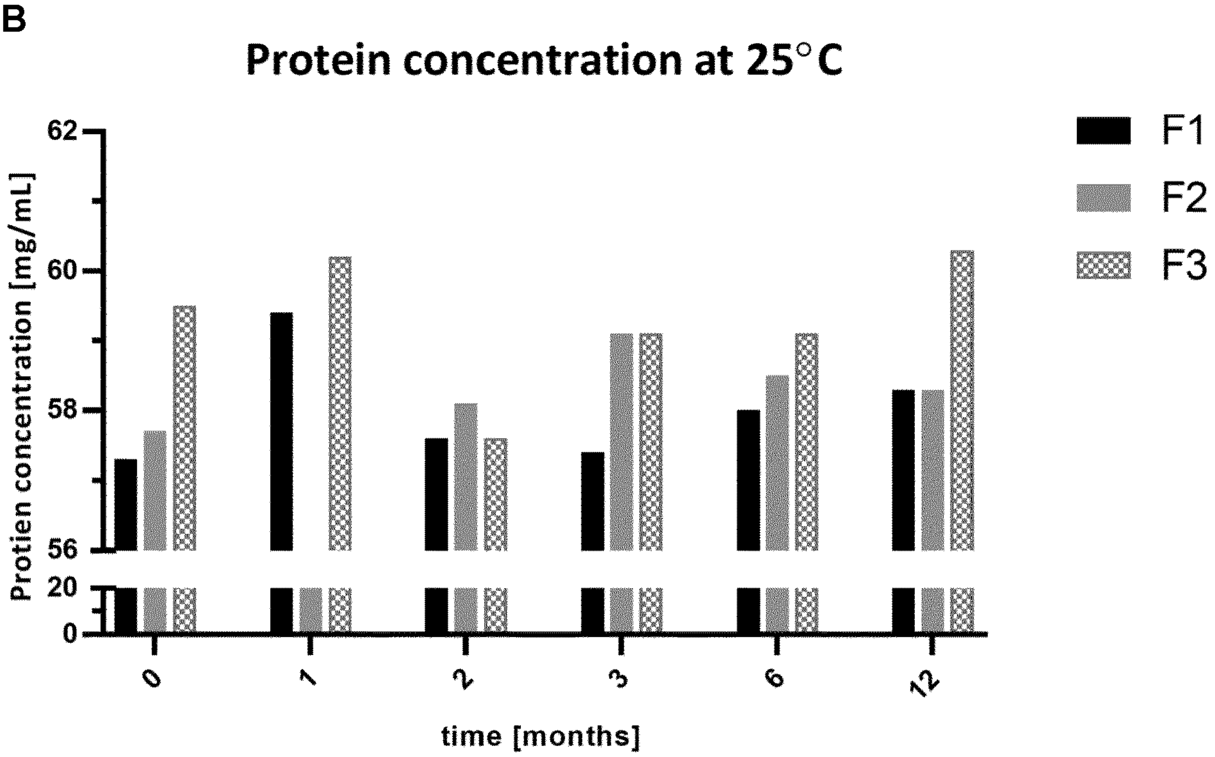


C

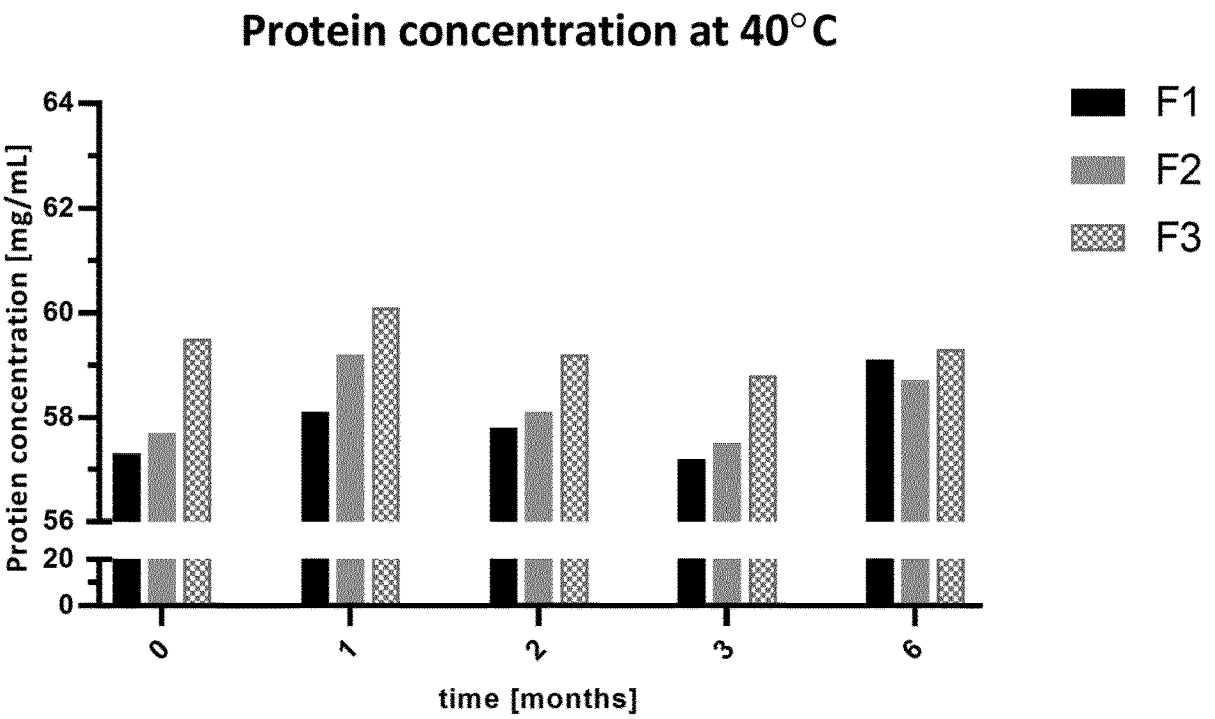

Figure 3

A

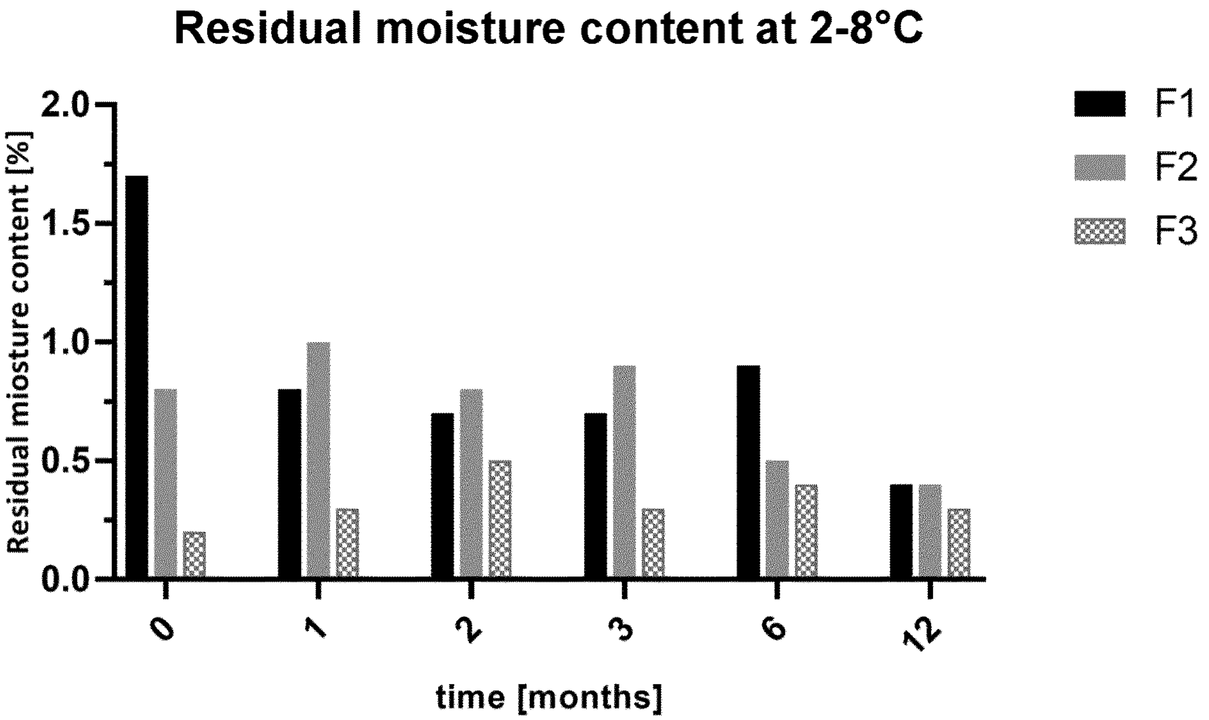
Residual moisture content at 2-8°C
Residual miosture content [%]
2.0
1.5
1.0
0.5
0.0
0
1
2
3
6
12
time [months]
F1
F2
F3

B

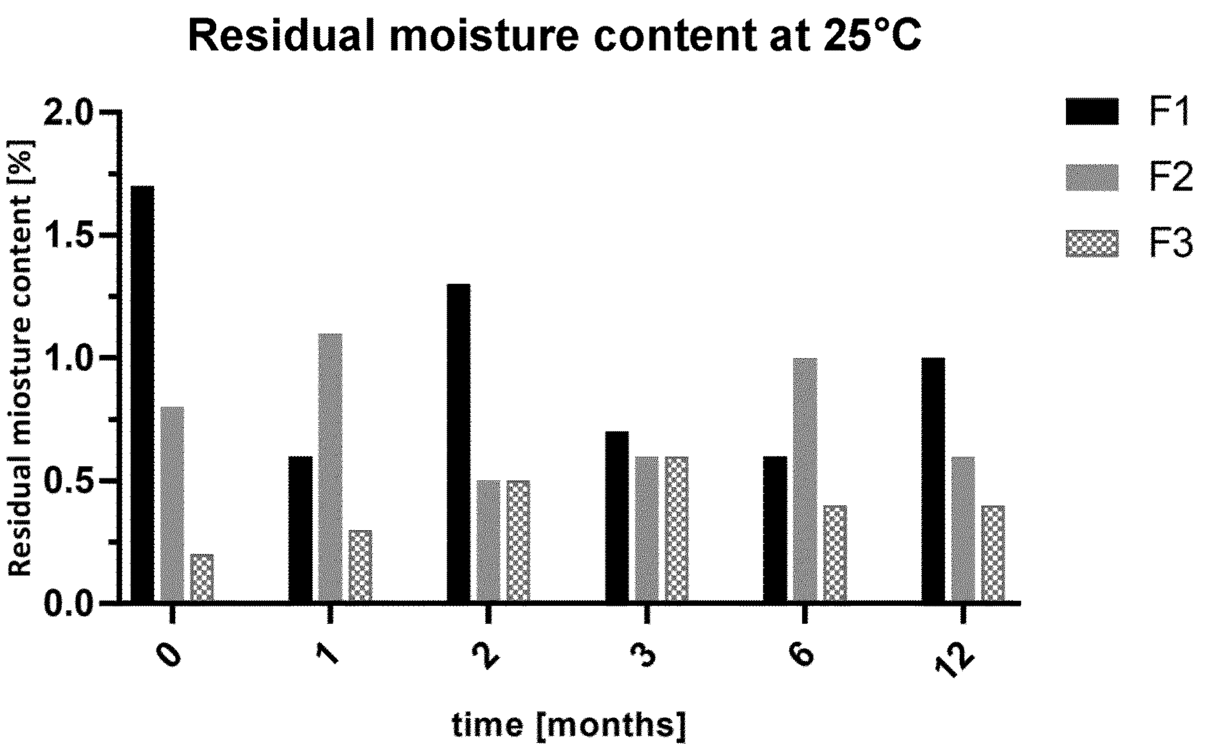
Residual moisture content at 25°C
Residual miosture content [%]
2.0
1.5
1.0
0.5
0.0
0
1
2
3
6
12
time [months]
F1
F2
F3

Figure 3 - continued

C

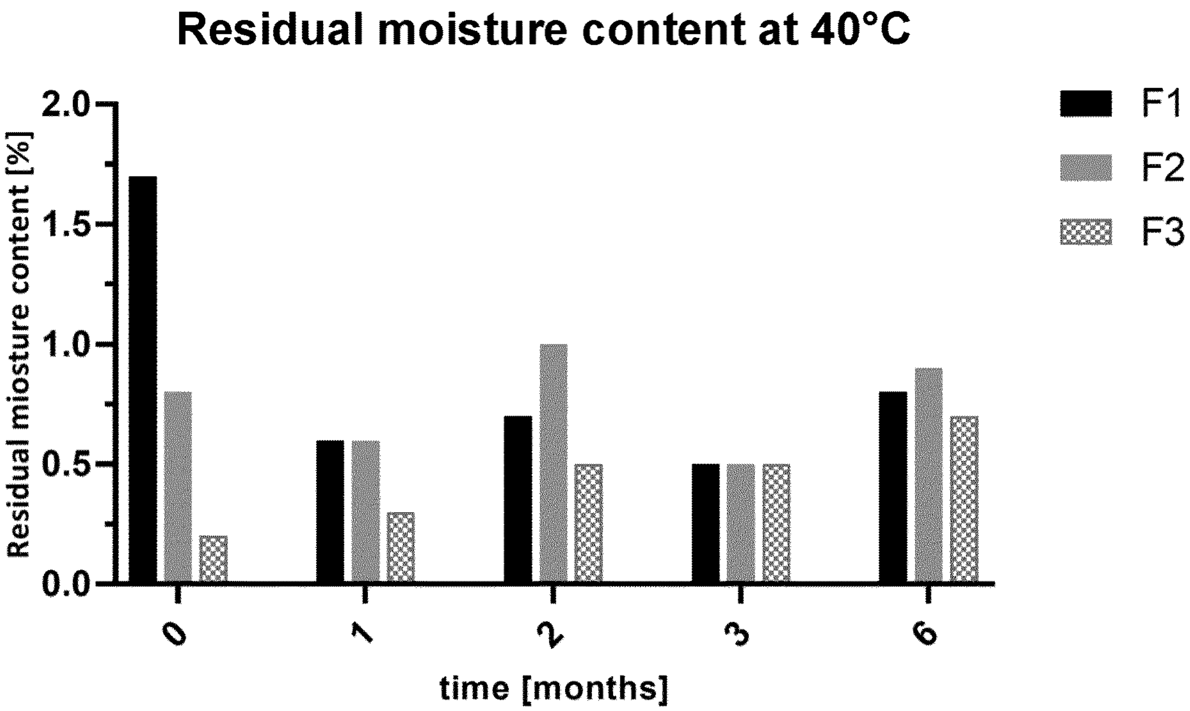


Figure 4

A

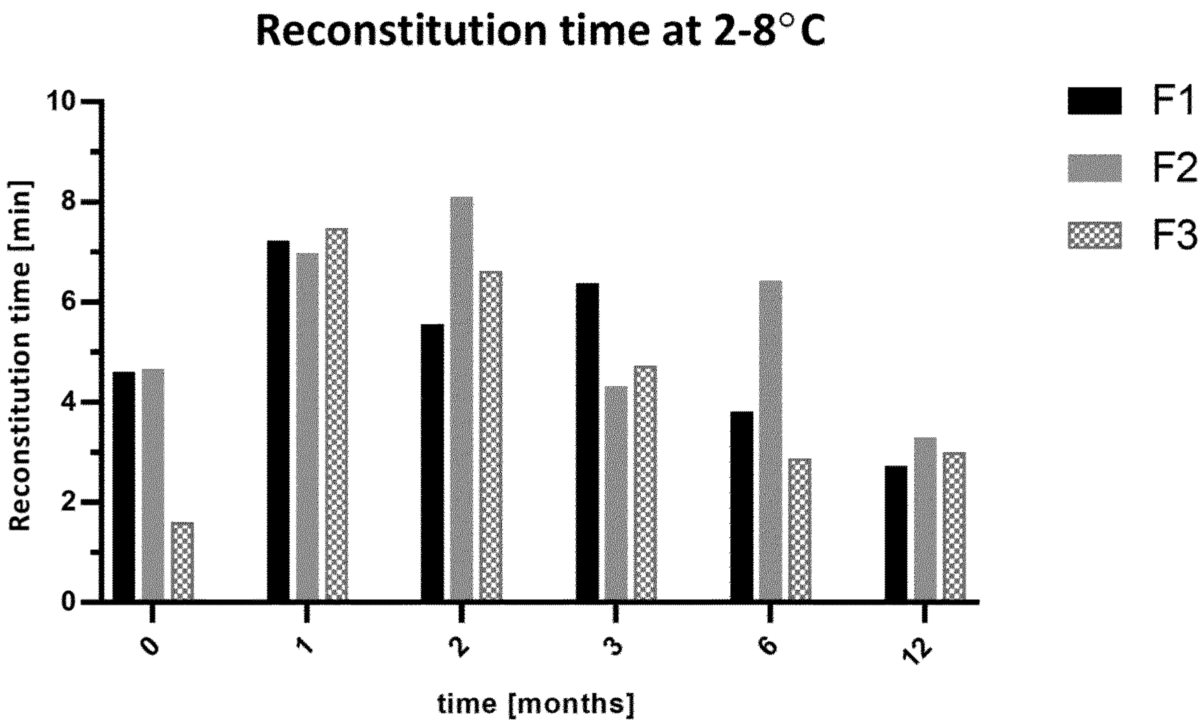

Figure 4 - continued

B

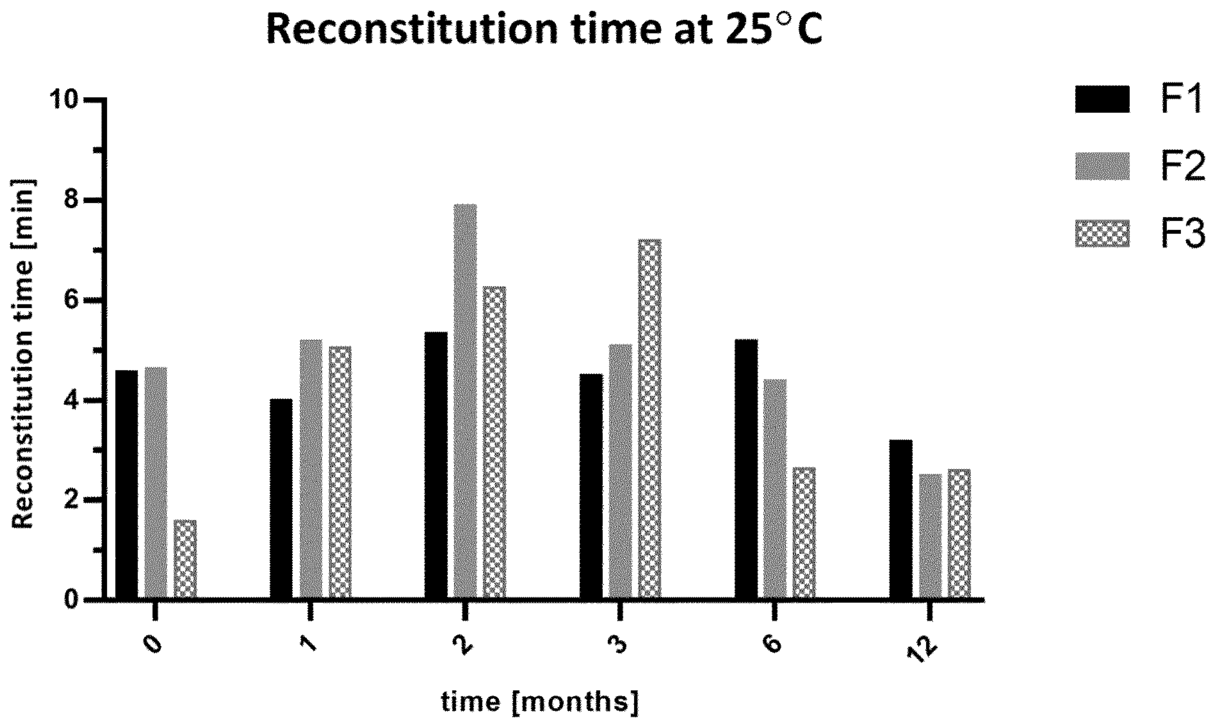


C

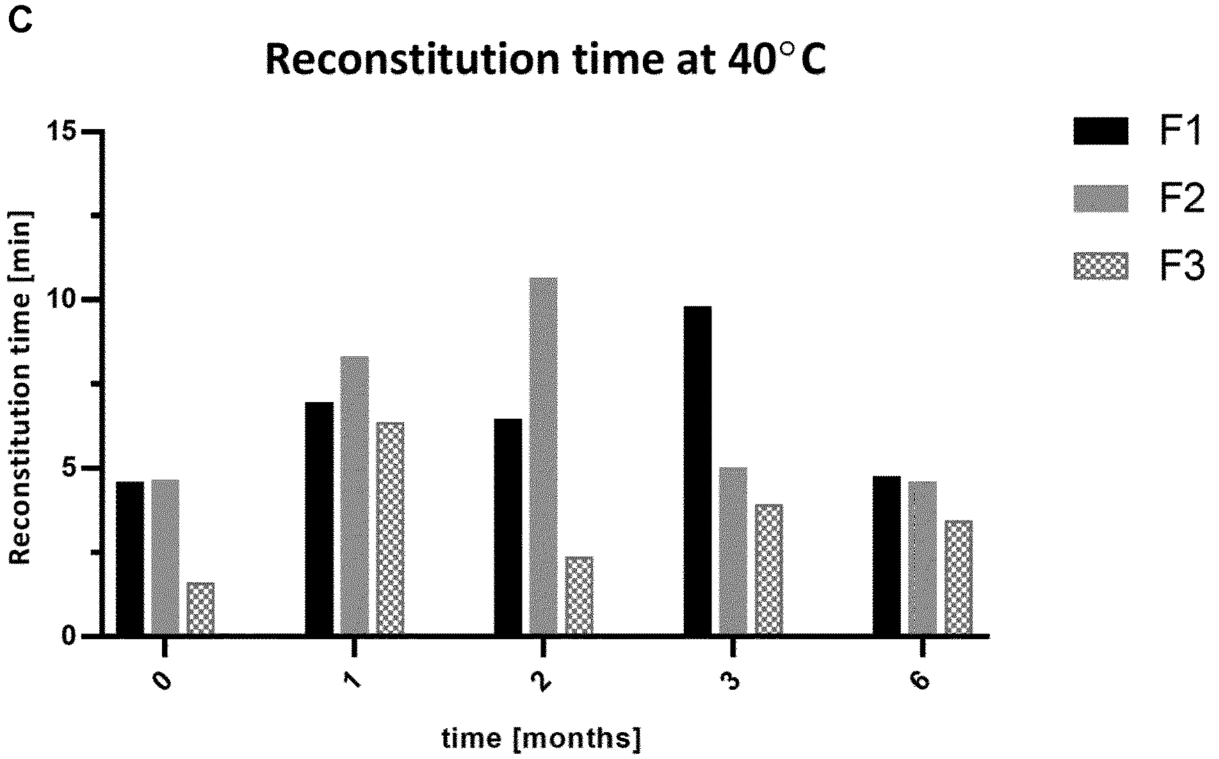

Figure 5

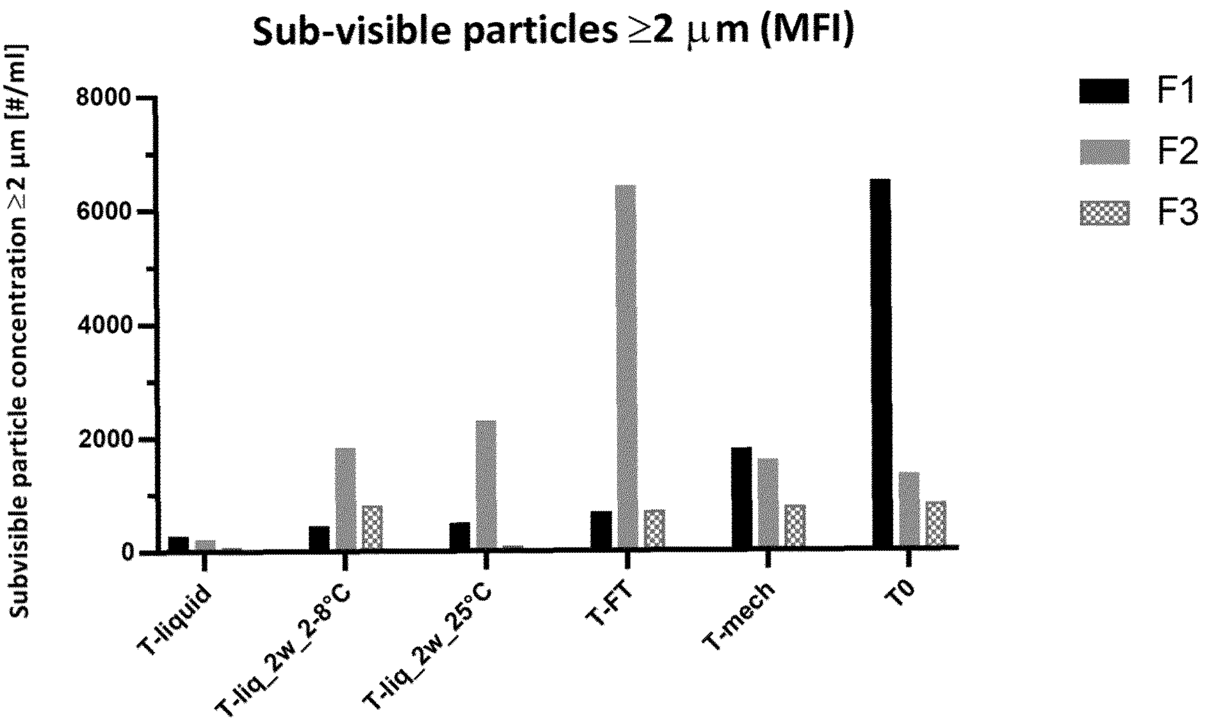
Sub-visible particles ≥2 μm (MFI)
Subvisible particle concentration ≥2 µm [#/ml]
8000
6000
4000
2000
0
T-liquid
T-liq_2w_2-8°C
T-liq_2w_25°C
T-FT
T-mech
T0
F1
F2
F3

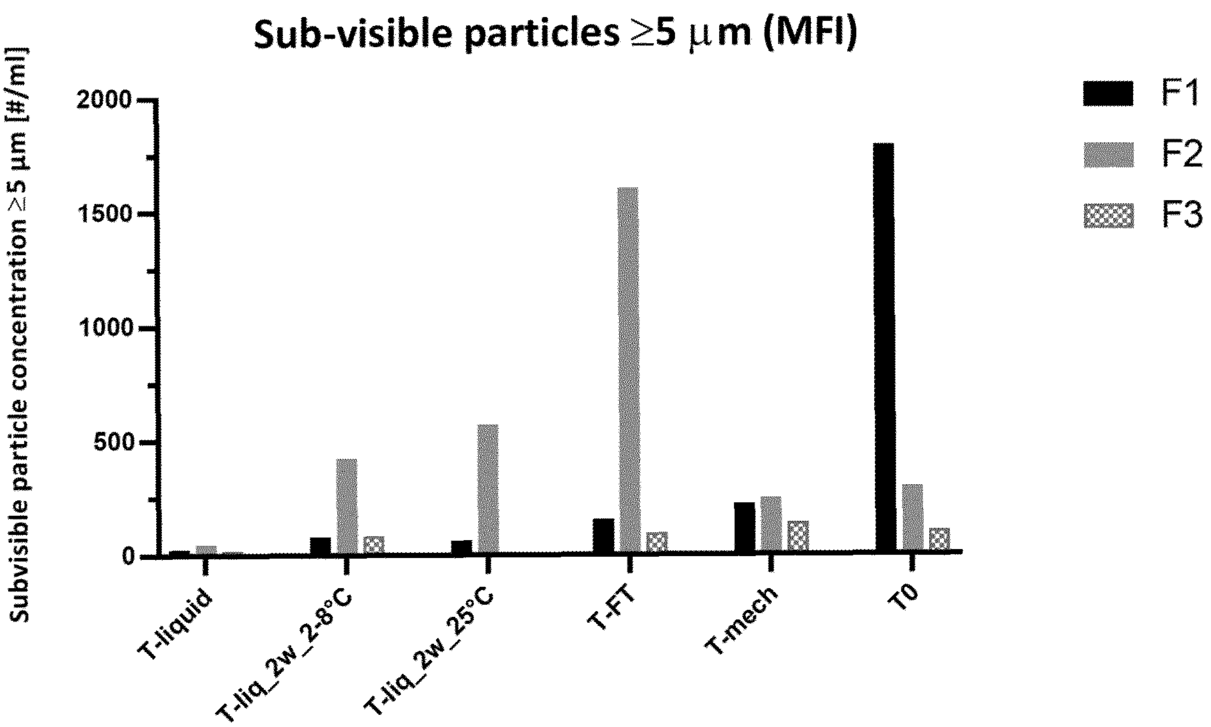
Sub-visible particles ≥5 μm (MFI)
Subvisible particle concentration ≥5 µm [#/ml]
2000
1500
1000
500
0
T-liquid
T-liq_2w_2-8°C
T-liq_2w_25°C
T-FT
T-mech
T0
F1
F2
F3

**Figure 5 - continued**

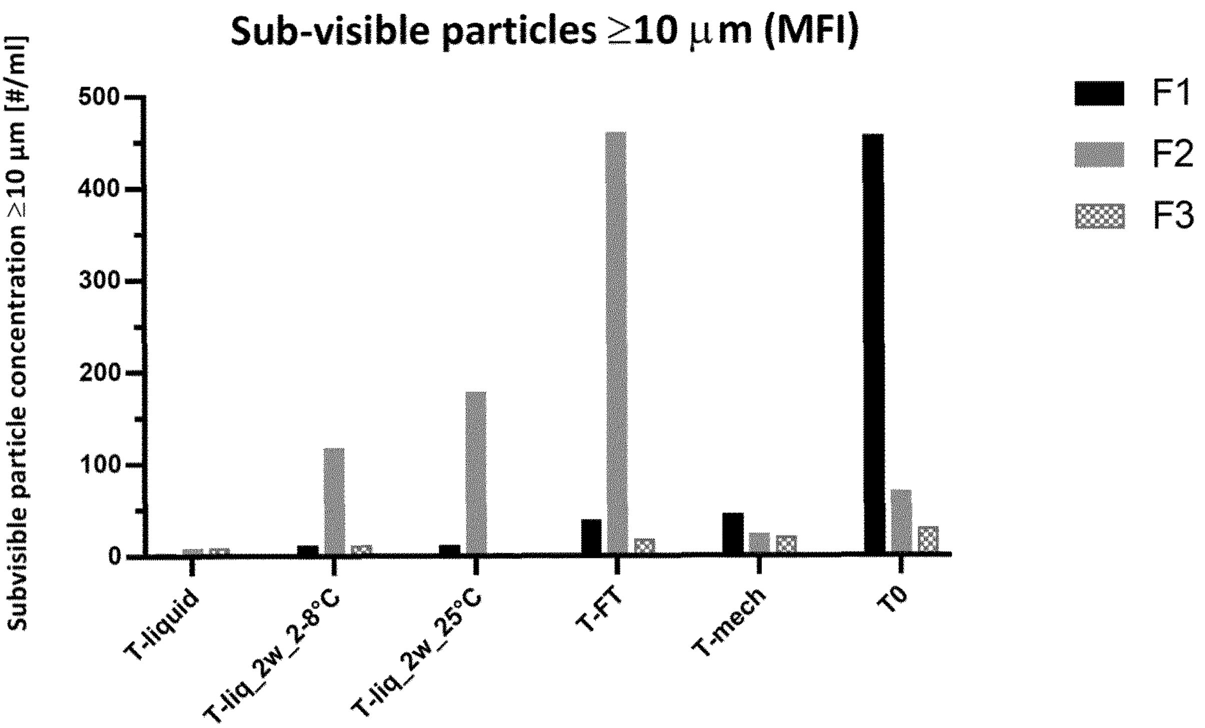


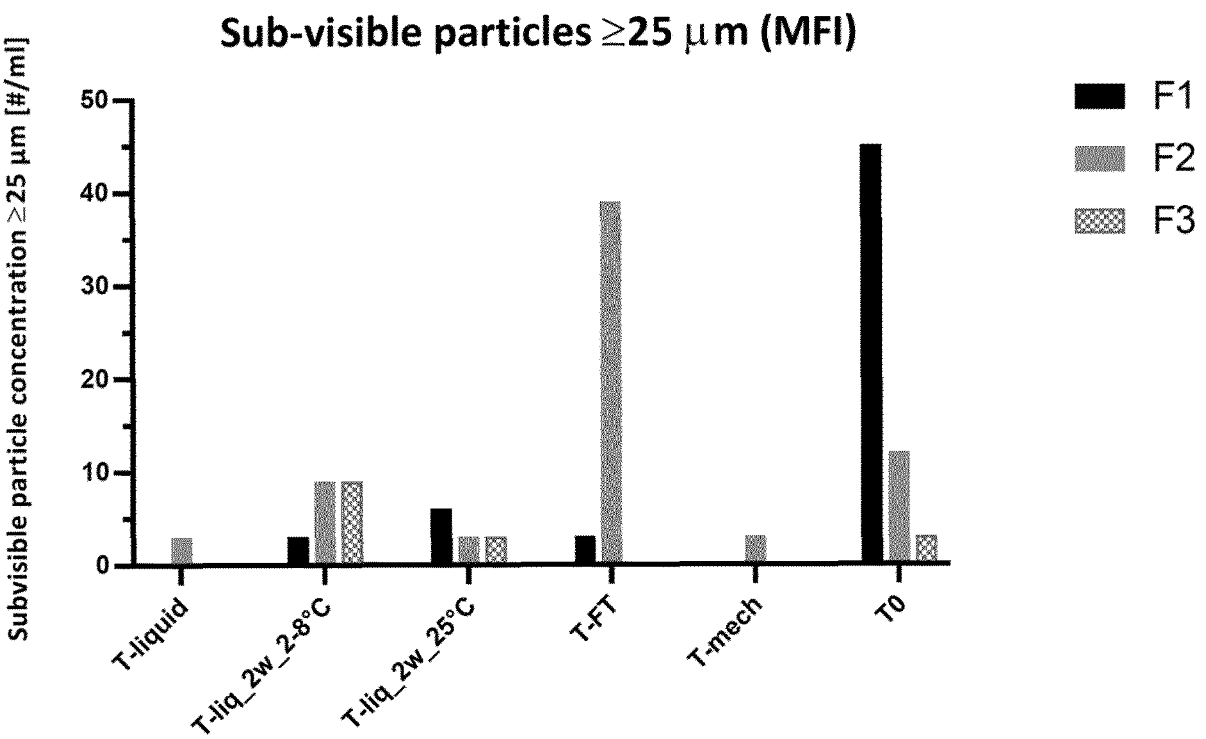

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *WHO Drug Information*, 2008, vol. 22 (4)